(19) Europäisches Patentamt
European Patent Office
Office européen des brevets

(11) Publication number : 0 572 110 A2

# (12) EUROPEAN PATENT APPLICATION

(21) Application number : 93302218.8

(22) Date of filing : 23.03.93

(51) Int. Cl.[5] : A61K 31/425, A61K 37/66

(30) Priority : 23.04.92 US 872549

(43) Date of publication of application :
01.12.93 Bulletin 93/48

(84) Designated Contracting States :
BE CH DE ES FR GB IT LI SE

(71) Applicant : CLINTEC NUTRITION COMPANY
Three Parkway North, Suite 500
Deerfield, Illinois 60015 (US)

(72) Inventor : Goldberg, Dennis I.
3 Newhaven Drive
Hawthorn Woods, IL 60047 (US)
Inventor : White, Randy D.
3810 Tecoma Drive
Crystal Lake, IL 60012 (US)
Inventor : Wilson, Daniel M.
868 Carol Avenue
Woodstock, IL 60098 (US)

(74) Representative : MacGregor, Gordon
ERIC POTTER & CLARKSON St. Mary's Court
St. Mary's Gate
Nottingham, NG1 1LE (GB)

## (54) Method of reducing or preventing toxicity associated with antiretroviral therapy by stimulation of the intracellular gluthatione level.

(57) The present invention provides a method for reducing, or preventing, the toxicity associated with antiretroviral therapy. To this end, when a glutathione intracellular stimulator is administered to a patient receiving antiretroviral therapy, the toxicity associated with such an agent is reduced. In a preferred embodiment, the antiretroviral therapy comprises AZT and DDI. Preferably, the composition for stimulating the intracellular glutathione levels comprises L-2-oxothiazolidine-4-carboxylate. The present invention also provides a method for treating a patient who is HIV positive, or who has AIDS, comprising the step of administering to the patient an antiretroviral therapy and administering to the patient an intracellular glutathione level in a sufficient amount to reduce the toxicity of the antiretroviral therapy.

EP 0 572 110 A2

## BACKGROUND OF THE INVENTION

The present invention relates generally to the treatment of disease states in patients. More specifically, the present invention relates to the treatment of acquired immunodeficiency syndrome (AIDS)

Presently, 3'-Azido-3'-deoxythymidine (AZT) is the primary therapy for the treatment of AIDS. AZT is an antiretroviral drug that is active against human immunodeficiency virus (HIV). In this regard, AZT is an inhibitor of the in vitro replication of some retroviruses, including HIV. AZT is marketed by the Burroughs Wellcome Company under the name Retrovir™. Retrovir is the brand name for Zidovudine (formerly called azidothymidine (AZT)). See Physician Desk Reference, 44th Edition, 1990, pg. 799.

Recently, attention has been focussed on another potential treatment for AIDS using DDI. DDI is also an antiretroviral drug.

Unfortunately, AZT has a significant dose dependent toxicity. In fact, the 1990 PDR bears the following statement:

Warning

Therapy with Retrovoir (Zidovudine) is often associated with hematologic toxicity including granulocytopenia and severe anemia requiring transfusions (see warnings).

AZT related bone marrow toxicity limits the utility of the drug. See, Thompson et al, "Hematologic Toxicity of AZT and ddC Administered as Single Agent and in Combination to Rats and Mice", Fundamental and Applied Toxicology, 17, 159-176 (1991). Although the basis of the toxicity is not completely understood, some evidence suggests a metabolite of AZT, 3'-amino-3'-deoxythymidine (AMT), may be at least partially responsible. See, Cretton, "Catabolism of 3'-Azido-3'-deoxythymidine in Hepatocytes in Liver Microsomes, with Evidence of Formation of 3'-Amino-3'-deoxythymidine, a Highly Toxic Catabolite for Human Bone Marrow Cells", Molecular Pharmacology, 39:258-266. It has been reported that some thiols, including reduced glutathione, can reduce AZT to AMT in vivo. See, Handlon et al, "Thiol Reduction of 3'-Azidothymidine to 3'-Aminothymidine: Kinetics and Biomedical Implications", Pharmaceutical Research, Vol. 5, No. 5, 1988.

Additionally, long term administration of AZT can cause mitochondrial myotoxicity. See, Lamperth et al, "Abnormal Skeletal and Cardiac Muscle Mitochondria Induced by Zidovudine (AZT) in Human Muscles In Vitro and in Animal Model", Laboratory Investigation, 1991, 65:742. This results in a myopathy that develops after a mean period of 12 months of therapy. Id.

Further adverse reactions have been reported with AZT. Additionally, DDI has been reported to also cause a variety of severe adverse reactions.

Due to the dose dependent toxicity of AZT and DDI the effectiveness of these drugs as therapies in treating AIDS has been limited.

## SUMMARY OF THE INVENTION

The present invention provides a method for reducing, or preventing, the toxicity associated with antiretroviral therapy. In this regard, the inventors have surprisingly found that when a glutathione intracellular stimulator is administered to a patient receiving antiretroviral therapy, the toxicity associated with such an agent is reduced. The present invention thereby provides a method for reducing the toxicity associated with antiretroviral therapy comprising the step of administering to a patient receiving antiretroviral therapy a therapeutically effective amount of a composition that stimulates the intracellular synthesis of glutathione.

In a preferred embodiment, the antiretroviral therapy comprises AZT or DDI. Preferably, the composition for stimulating the intracellular glutathione levels comprises L-2-oxothiazolidine-4-carboxylate.

The present invention also provides a method for treating a patient who is HIV positive, or who has AIDS, comprising the steps of administering to the patient an antiretroviral therapy and administering to the patient an intracellular glutathione stimulator in a sufficient amount to reduce the toxicity of the antiretroviral therapy.

An advantage of the present invention is that it reduces, or prevents, the toxicity associated with antiretroviral therapy. This allows compounds such as AZT to be more effectively used in the treatment of AIDS.

An additional advantage of the present invention is that it allows antiretroviral therapy to be administered to a patient who is HIV positive, but not expressing the symptoms of AIDS or ARC, prophylactically, without at least some of the severe side effects heretofore caused by the therapy, e.g., hematologic toxicity.

Additional features and advantages of the present invention are described in, and will be apparent from, the detailed description of the presently preferred embodiments.

## DETAILED DESCRIPTION OF THE PRESENTLY PREFERRED EMBODIMENTS

The treatment of HIV and/or AIDS infection with antiretroviral therapy is limited due to the severe toxicity

associated with such agents. For example, AZT causes a dose dependent hemotoxicity. DDI also induces severe life threatening adverse side effects. The toxicity of such antiretroviral agents is dose dependent thereby limiting the therapeutic approaches that can be taken with such compounds.

The inventors have surprisingly found that an intracellular glutathione stimulator when administered to a patient taking antiretroviral compounds will reduce or prevent toxicity associated with the antiretroviral therapy. This is a surprising result in view of the fact that it had been reported that reduced glutathione, and other thiols, may reduce AZT to AMT. See, Handlon et al, supra.

This belief, coupled with the evidence suggesting that AMT may be at least partially responsible for the bone marrow toxicity of AZT (see, Cretton, supra) would lead one away from such a combination. Surprisingly, the inventors have found that an intracellular glutathione stimulator will reduce the toxicity of AZT. As set forth in detail below in the experiment described in the example, the inventors have specifically found that L-2-oxothiazolidine-4-carboxylate will reduce the toxicity of AZT.

L-2-oxothiazolidine-4-carboxylate is subjected to the action of 5-oxo-L-prolinase in the presence of adenosine triphosphate to produce S-carboxyl cysteine. S-carboxyl cysteine is then decarboxylated to produce cysteine. Cysteine is then metabolized to provide glutathione. See, U.S. Patent Nos.: 4,335,210; 4,434,158; 4,438,124; 4,665,082; and 4,647,571, the disclosures of which are incorporated herein by reference.

The inventors also believe that other substrates that stimulate intracellular glutathione synthesis can also be utilized to reduce, or eliminate, the toxicity of antiretroviral drugs, such as AZT and DDI. To this end, the inventors believe that glutathione esters, as well as other thiazolidine-4-carboxylate analogs, that are converted intracellularly the glutathione can be utilized.

Such a glutathione ester, for example, can have the structure:

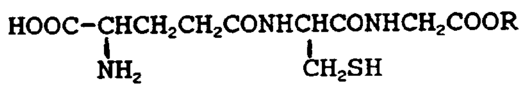

wherein: R is an alkyl group containing 1 to 10 carbon atoms. Preferably, the methyl and ethyl glutathione esters are used. Glutathione esters are disclosed in U.S. Patent No. 4,710,489, the disclosure of which is incorporated herein by reference.

Further, the inventors postulate that proteins enriched in cysteine, that will also stimulate the intracellular glutathione synthesis, can be utilized. Such proteins, if they have sufficiently high cysteine levels and are administered in sufficiently high quantities, it is believed, can provide the necessary intracellular glutathione stimulation. For example, the following proteins have a high cysteine content: whey (2%); egg white (2.5%); serum albumin (5.5%); and lactalbumin (5.8%).

Pursuant to the present invention, the composition of the present invention can either be administered parenterally or enterally. Enterally, the composition can comprise a pill including, for example, L-2-oxothiazolidine-4-carboxylate that is administered to a patient either contemporaneously with the antiretroviral drug or on the same days. Likewise, the composition can include an enteral solution that includes the intracellular glutathione stimulator. It is even envisioned that the composition can be part of the patients' diet.

Parenterally, the composition can, for example, include approximately 3% to about 6%, by weight, L-2-oxothiazolidine-4-carboxylate in a buffer solution such as phosphate buffer. Of course, parenterally, the composition can be administered as a bolus or as an additive to an IV solution.

By way of example, and not limitation, an example of the present invention will now be given.

## EXAMPLE

To evaluate the hematologic response (peripheral blood and bone marrow), mice were treated by gavage twice daily for 13.5 days with 3'-azido-3'-deoxythymidine (AZT) while being administered L-2-oxothiazolidine-4-carboxylate (Procysteine™) orally with the feed.

L-2-oxothiazolidine-4-carboxylate (Procysteine™), was provided by Clintec Nutrition Company, Deerfield, Illinois. Diet (MEAL) containing 1.0% (wt/wt) Procysteine™ was formulated. The control diet consisted of MEAL containing no Procysteine™.

3'-azido-3'-deoxythymidine (AZT) was provided by Sigma Chemical Company, St. Louis, Missouri. AZT was formulated as a suspension at 2.5 and 25 mg/mL in sterile water containing 0.5% (wt/vol) methylcellulose. AZT formulations were dispensed into amber glass bottles.

0.5% (wt/vol) methylcellulose in sterile water was used as the vehicle control. The vehicle control was dispensed and handled in a similar fashion as the positive control article.

The control diet and diet containing Procysteine™ were stored at room temperature in sealed containers

in the study room. While in a fume hood, the diet containing Procysteine™ was weighed and dispensed. In a fume hood, AZT was weighed, formulated into a suspension and dispensed into bottles.

The vehicle control and AZT formulations were stored refrigerated at 5±3°C. At each gavage dosing period, one bottle of each of the formulations was removed from the refrigerator approximately 30-60 minutes before dosing and maintained with continual stirring using a magnetic stir bar until the completion of dosing. While in a fume hood, mice were treated by gavage twice a day, starting on study day 3.

Sixty male and sixty female mice from Charles River Laboratories, Portage, Michigan, were studied. Mice were approximately 7-8 weeks old at the start of AZT dosing. On study day 0, the weight of the mice was as follows: males: 17-30 g; and females: 14-25 g. Only mice showing no signs of clinical illness were used in this study. The mice were from a colony which was raised and certified by the vendor to be free of adventitious pathogens as indicated upon receipt.

The experimental design for treating the animals was as shown in Table 1. Three days prior to the initiation of AZT treatment (i.e., on study days 0, 1, and 2), mice in groups 2, 5, and 6 were fed the diet containing 1.0% (wt/wt) Procysteine™. Mice in groups 1, 3, and 4 were fed the control diet.

Treatment with these diets was continued on study days 3-16.

Starting on study day 3, mice in groups 1 and 2 were treated by oral gavage with a vehicle control (0.5% methyl-cellulose in distilled water) or, in groups 3, 4, 5, and 6, with a positive control article (AZT at 50 or 500 mg/kg body weight/day), as appropriate. Mice were weighed before the first treatment every day to calculate dosage. Mice were treated by gavage twice daily, starting at approximately 9 a.m. and 3 p.m., receiving 1/2 of the daily dose at each treatment. At each time point, mice were treated using a volume of approximately 10 mL/kg body weight.

Animals were weighed (not fasted) and sacrificed on study day 17. At this time, blood was collected for hematology evaluation, and bone marrow was collected for bone marrow and hematopoietic progenitor cell assays.

**Table 1.**

**Scheme for Treating Mice with AZT and Procysteine™**

| Group No. | AZT Daily Dosage[1] (mg/kg) (starting day 3) | Procysteine™ (% of Diet) (starting Day 0) | No. Mice/Group (male/female) |
|---|---|---|---|
| 1 | 0 | 0 | 10/10 |
| 2 | 0 | 1.0 | 10/10 |
| 3 | 50 | 0 | 10/10 |
| 4 | 500 | 0 | 10/10 |
| 5 | 50 | 1.0 | 10/10 |
| 6 | 500 | 1.0 | 10/10 |

[1]AZT was given by gavage at 1/2 the daily dosage, twice daily for 14 days in a volume of 10 mL/kg body weight. AZT was formulated at 2.5 and 25 mg/mL for the 50 and 500 mg/kg body weight treatments, respectively.

Mice received Certified Rodent Diet - MEAL (Ralston Purina Co., St. Louis, Missouri). Three days prior to the initiation of AZT, mice in groups 2, 5, and 6 were placed on diet containing 1.0% (wt/wt) Procysteine™. The diet was put into dishes placed in each cage. Food consumption was monitored on a daily basis for all animals within a block upon the initiation of Procysteine™ feeding to animals within that block.

Deionized drinking water was provided ad libitum through demand-controlled valves in each cage.

To accommodate the necropsy procedure, animals were assigned to 1 of 5 blocks (24 mice/block) with

initiation of treatment occurring on 5 consecutive days (i.e., study day 0 for block 2 is one day after study day 0 for block 1, and so on) (see Table 2). Male mice were numbered from 1 through 60, female mice from 61 through 120, according to their body weight on study day 0 for block 1. For a given sex of animal, the heavier the animal, the smaller the number assigned. The assignment kept constant the sum of the numbers (ranks) across the 2 animals in each group of a block for a given sex. Those sums differed from block to block by an increment of 4. Such a small difference due to blocks was controlled in the statistical analysis. The main goal of this systematic assignment was to maintain equal weights across groups. The assignment of animals to treatment groups (5 blocks) is given in Table 2.

Mice were treated and necropsied according to block, in ascending group order; per block, males were treated first, followed by females. For example, Procysteine™ treatment started for block 1 on study day 0; the next day was study day 1 for block 1 and study day 0 for block 2, and so on. At any given gavage period (a.m. or p.m.), all males and females of a given block were treated before mice from the next block were started.

**Table 2. Systematic Assignment of Animals**

| Group | Sex | 1 | 2 | 3 | 4 | 5 |
|---|---|---|---|---|---|---|
| 1 | M | 01, 52 | 13, 44 | 25, 36 | 07, 58 | 19, 50 |
| | F | 61, 112 | 73, 104 | 85, 96 | 67, 118 | 79, 110 |
| 2 | M | 12, 41 | 24, 33 | 06, 55 | 18, 47 | 30, 39 |
| | F | 72, 101 | 84, 93 | 66, 115 | 78, 107 | 90, 99 |
| 3 | M | 21, 32 | 03, 54 | 15, 46 | 27, 38 | 09, 60 |
| | F | 81, 92 | 63, 114 | 75, 106 | 87, 98 | 69, 120 |
| 4 | M | 02, 51 | 14, 43 | 26, 35 | 08, 57 | 20, 49 |
| | F | 62, 111 | 84, 103 | 86, 95 | 68, 117 | 80, 109 |
| 5 | M | 11, 42 | 23, 34 | 05, 56 | 17, 48 | 29, 40 |
| | F | 71, 102 | 83, 94 | 65, 116 | 77, 108 | 89, 100 |
| 6 | M | 22, 31 | 04, 53 | 16, 45 | 28, 37 | 10, 59 |
| | F | 82, 91 | 64, 113 | 76, 105 | 88, 97 | 70, 119 |
| Sum of | M | 53 | 57 | 61 | 65 | 65 |
| Ranks | F | 173 | 177 | 181 | 185 | 189 |

At approximately 3:00 p.m. on study day 0, animals were placed on a measured amount of diet (at least approximately 11 g/day) containing 0 or 1% Procysteine™, as appropriate. Feed was administered in stainless steel dishes placed in each cage. At approximately 7:00 a.m. on each of the succeeding days, the feed containers were removed, weighed, cleaned, and fresh diet added; containers were returned to the cages at approximately 3:00 p.m. each day except study day 17. On study days 3-16, each animal was treated by gavage twice daily, starting at approximately 9:00 a.m. and 3:00 p.m., with approximately 10 mL/kg body weight of the vehicle control, or the positive control (AZT) at 50 or 500 mg/kg body weight.

Starting on study day 0, body weights were recorded daily.

Each animal was observed approximately 1 hour after being treated by gavage.

On study day 17, the animals were anesthetized with methoxyflurane (in a bell jar). When deeply anesthetized, blood was obtained by cardiac puncture and transferred for hematological analysis into tubes containing an EDTA solution.

Bone marrow smear slides were prepared by extracting a small quantity of bone marrow from a femur using a small brush previously dipped in mouse serum and then brushing the marrow onto the slides. The bone marrow slides were dipped (five times for approximately one second each time) into Diff-Quik® fixative and allowed to air dry. Fixed slides were stained and evaluated microscopically.

The following hemotologic assays were conducted.

Leukocyte count
Erythrocyte count
Total hemoglobin concentration
Hematocrit

Mean corpuscular volume (MCV)
Mean corpuscular hemoglobin (MCH)
Mean corpuscular hemoglobin concentration (MCHC)
Platelet count
Reticulocyte count
Differential leukocyte count

Populations of bone marrow colony forming cells were evaluated. Bone marrow was collected and the stem cell assay conducted, as follows: one femur per mouse was cut at the distal and proximal ends. Using a syringe and 25 gauge needle, 5 mL of IMDM media was flushed through the femur into a collection tube (2.5 mL flushed into one end, then 2.5 ml into the other end). The tube was washed using Iscove's Modified Dulbecco's Media containing 20% fetal bovine serum and the cell concentration was adjusted to the desired count for the colony assay.

In triplicate, the marrow cells were put into a mixture of methylcellulose and plated into 35 mm diameter dishes. The dishes were incubated approximately 2 weeks (37°C, 5% $CO_2$, 100% relative humidity) then manually scored under a microscope for myeloid, erythroid, and mixed colonies.

Data generated included the following:

| | |
|---|---|
| Body weights: | Initial and daily |
| Hematology: | Blood collected prior to necropsy |
| Bone marrow: | Bone marrow count, Myeloid/Erythroid ratio, hematopoietic progenitor cell population |
| Food consumption: | Daily |

Statistical tests were performed at the $\alpha$ level of 0.01. Listing of the individual data and summary statistics (mean, standard error and sample size) was provided. The average body weight changes from study day 3 for study days 4-10 and 11-17 and average food consumption for study days 4-10 and 11-17 was analyzed. Since the groups consisted of various dosages of AZT and Procysteine™, appropriate dosage response models were established. Specifically, the interaction of AZT and Procysteine™ was evaluated.

Table 3: Body Weight Changes
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Weight Change Week 1 (g)** | | | | | | | | | | | | | |
| Male | | | | | | | | | | | | | |
| Mean | 1.1 | 1.4 | 1.1 | 0.8 | 1.3 | 1.0 | | | | | | | |
| Std. Err. | 0.2 | 0.3 | 0.3 | 0.2 | 0.3 | 0.2 | | | | | | | |
| N | 10 | 10 | 10 | 10 | 10 | 10 | | | | | | | |
| Female | | | | | | | | | | | | | |
| Mean | 1.0 | 1.1 | 1.0 | 0.6 | 1.0 | 0.9 | | | | | | | |
| Std. Err. | 0.2 | 0.1 | 0.1 | 0.2 | 0.2 | 0.2 | | | | | | | |
| N | 10 | 10 | 10 | 10 | 10 | 10 | | | | | | | |
| Combined | | | | | | | | | | | | | |
| Mean | 1.1 | 1.3- | 1.1-- | 0.7-# | 1.1-- | 1.0-- | - | - | - | - | - | - | - |
| Std. Err. | 0.1 | 0.2 | 0.2 | 0.1 | 0.2 | 0.1 | | | | | | | |
| N | 20 | 20 | 20 | 20 | 20 | 20 | | | | | | | |
| **Weight Change Week 2 (g)** | | | | | | | | | | | | | |
| Male | | | | | | | | | | | | | |
| Mean | 2.4 | 2.5 | 2.5 | 2.1 | 2.3 | 1.9 | | | | | | | |
| Std. Err. | 0.2 | 0.3 | 0.4 | 0.3 | 0.3 | 0.2 | | | | | | | |
| N | 10 | 10 | 10 | 10 | 10 | 10 | | | | | | | |
| Female | | | | | | | | | | | | | |
| Mean | 1.8 | 1.8 | 1.8 | 1.5 | 1.8 | 1.8 | | | | | | | |
| Std. Err. | 0.2 | 0.2 | 0.2 | 0.2 | 0.2 | 0.2 | | | | | | | |
| N | 10 | 10 | 10 | 10 | 9 | 10 | | | | | | | |
| Combined | | | | | | | | | | | | | |
| Mean | 2.1 | 2.2- | 2.2-- | 1.8-- | 2.1-- | 1.9-- | - | - | - | - | - | - | - |
| Std. Err. | 0.1 | 0.2 | 0.2 | 0.2 | 0.2 | 0.1 | | | | | | | |
| N | 20 | 20 | 20 | 20 | 19 | 20 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 4: Blood Assays

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| | | | | | | | AZT DR at % Procyst. | | | Procysteine DR at mg/kg AZT | | | |
|---|---|---|---|---|---|---|---|---|---|---|---|---|---|
| AZT | 0 mg/kg | 0 mg/kg | 50 mg/kg | 500 mg/kg | 50 mg/kg | 500 mg/kg | | | | | | | |
| Procysteine | 0.0% | 1.0% | 0.0% | 0.0% | 1.0% | 1.0% | 0.0 | 1.0 | DIF | 0 | 50 | 500 | DIF |
| | | | | WBC (x10**3/uL) | | | | | | | | | |
| Male | | | | | | | | | | | | | |
| Mean | 1.7 | 1.9 | 1.6 | 2.3 | 1.6 | 2.1 | | | | | | | |
| Std. Err. | 0.3 | 0.2 | 0.2 | 0.3 | 0.1 | 0.2 | | | | | | | |
| N | 10 | 10 | 10 | 10 | 10 | 10 | | | | | | | |
| .... | | | | | | | | | | | | | |
| Female | | | | | | | | | | | | | |
| Mean | 2.1 | 1.6 | 1.7 | 2.3 | 2.0 | 1.6 | | | | | | | |
| Std. Err. | 0.2 | 0.1 | 0.2 | 0.2 | 0.2 | 0.2 | | | | | | | |
| N | 10 | 9 | 10 | 10 | 9 | 10 | | | | | | | |
| Combined | | | | | | | | | | | | | |
| Mean | 1.9 | 1.7- | 1.7-- | 2.3-# | 1.8-- | 1.9-- | - | - | - | - | - | - | - |
| Std. Err. | 0.2 | 0.1 | 0.1 | 0.2 | 0.1 | 0.1 | | | | | | | |
| N | 20 | 19 | 20 | 20 | 19 | 20 | | | | | | | |
| | | | | RBC (x10**6/uL) | | | | | | | | | |
| Male | | | | | | | | | | | | | |
| Mean | 7.46 | 7.46 | 6.95 | 6.18 | 7.13 | 6.19 | | | | | | | |
| Std. Err. | 0.06 | 0.03 | 0.05 | 0.08 | 0.08 | 0.11 | | | | | | | |
| N | 10 | 10 | 10 | 10 | 10 | 10 | | | | | | | |
| .... | | | | | | | | | | | | | |
| Female | | | | | | | | | | | | | |
| Mean | 7.25 | 7.36 | 6.77 | 6.22 | 6.99 | 6.29 | | | | | | | |
| Std. Err. | 0.07 | 0.06 | 0.04 | 0.10 | 0.10 | 0.05 | | | | | | | |
| N | 10 | 9 | 10 | 10 | 9 | 10 | | | | | | | |
| Combined | | | | | | | | | | | | | |
| Mean | 7.36 | 7.41- | 6.86@# | 6.20@# | 7.07@# | 6.24@# | D | D | - | - | I | - | - |
| Std. Err. | 0.05 | 0.04 | 0.04 | 0.07 | 0.06 | 0.06 | | | | | | | |
| N | 20 | 19 | 20 | 20 | 19 | 20 | | | | | | | |

@ Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 4: Blood Assays

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

HGB (g/L)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Male** | | | | | | | | | | | | | |
| Mean | 13.2 | 13.2 | 12.6 | 11.5 | 13.0 | 11.5 | | | | | | | |
| Std. Err. | 0.1 | 0.1 | 0.1 | 0.2 | 0.1 | 0.2 | | | | | | | |
| N | 10 | 10 | 10 | 10 | 10 | 10 | | | | | | | |
| .... | | | | | | | | | | | | | |
| **Female** | | | | | | | | | | | | | |
| Mean | 13.0 | 13.0 | 12.5 | 11.7 | 12.9 | 11.7 | | | | | | | |
| Std. Err. | 0.1 | 0.1 | 0.1 | 0.2 | 0.2 | 0.2 | | | | | | | |
| N | 10 | 9 | 10 | 10 | 9 | 10 | | | | | | | |
| **Combined** | | | | | | | | | | | | | |
| Mean | 13.1 | 13.1- | 12.5@# | 11.6@# | 12.9-- | 11.6@# | D | D | - | - | I | - | - |
| Std. Err. | 0.1 | 0.1 | 0.1 | 0.1 | 0.1 | 0.1 | | | | | | | |
| N | 20 | 19 | 20 | 20 | 19 | 20 | | | | | | | |

HCT (%)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Mean | 37.4 | 37.0 | 36.0 | 33.0 | 36.8 | 33.2 | | | | | | | |
| Std. Err. | 0.4 | 0.3 | 0.3 | 0.5 | 0.5 | 0.7 | | | | | | | |
| N | 10 | 10 | 10 | 10 | 10 | 10 | | | | | | | |
| .... | | | | | | | | | | | | | |
| **Female** | | | | | | | | | | | | | |
| Mean | 36.1 | 35.9 | 34.4 | 32.1 | 35.2 | 32.5 | | | | | | | |
| Std. Err. | 0.4 | 0.4 | 0.3 | 0.5 | 0.5 | 0.5 | | | | | | | |
| N | 10 | 9 | 10 | 10 | 9 | 10 | | | | | | | |
| **Combined** | | | | | | | | | | | | | |
| Mean | 36.8 | 36.5- | 35.2@# | 32.5@# | 36.0-- | 32.8@# | D | D | - | - | - | - | - |
| Std. Err. | 0.3 | 0.2 | 0.3 | 0.4 | 0.4 | 0.4 | | | | | | | |
| N | 20 | 19 | 20 | 20 | 19 | 20 | | | | | | | |

@ Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 4: Blood Assays

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **MCV (fL)** | | | | | | | | | | | | | |
| Male | | | | | | | | | | | | | |
| Mean | 51 | 51 | 52 | 54 | 52 | 54 | | | | | | | |
| Std. Err. | 0 | 0 | 0 | 0 | 0 | 0 | | | | | | | |
| N | 10 | 10 | 10 | 10 | 10 | 10 | | | | | | | |
| Female | | | | | | | | | | | | | |
| Mean | 50 | 49 | 51 | 52 | 51 | 52 | | | | | | | |
| Std. Err. | 0 | 0 | 0 | 0 | 0 | 1 | | | | | | | |
| N | 10 | 9 | 10 | 10 | 9 | 10 | | | | | | | |
| Combined | | | | | | | | | | | | | |
| Mean | 51 | 50- | 52@# | 53@# | 52@# | 53@# | I | I | - | - | - | - | - |
| Std. Err. | 0 | 0 | 0 | 0 | 0 | 0 | | | | | | | |
| N | 20 | 19 | 20 | 20 | 19 | 20 | | | | | | | |
| **MCH (pg)** | | | | | | | | | | | | | |
| Male | | | | | | | | | | | | | |
| Mean | 17.6 | 17.7 | 18.2 | 18.6 | 18.3 | 18.6 | | | | | | | |
| Std. Err. | 0.1 | 0.1 | 0.1 | 0.2 | 0.1 | 0.1 | | | | | | | |
| N | 10 | 10 | 10 | 10 | 10 | 10 | | | | | | | |
| Female | | | | | | | | | | | | | |
| Mean | 18.0 | 17.6 | 18.4 | 18.8 | 18.4 | 18.6 | | | | | | | |
| Std. Err. | 0.2 | 0.1 | 0.1 | 0.2 | 0.2 | 0.2 | | | | | | | |
| N | 10 | 9 | 10 | 10 | 9 | 10 | | | | | | | |
| Combined | | | | | | | | | | | | | |
| Mean | 17.8 | 17.7- | 18.3@# | 18.7@# | 18.3@# | 18.6@# | I | I | - | - | - | - | - |
| Std. Err. | 0.1 | 0.1 | 0.1 | 0.1 | 0.1 | 0.1 | | | | | | | |
| N | 20 | 19 | 20 | 20 | 19 | 20 | | | | | | | |

@ Indicates Difference From Group 1 : 0 mg/kg AZT, 0.0% Procysteine

\# Indicates Difference From Group 2 : 0 mg/kg AZT, 1.0% Procysteine

\- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 4: Blood Assays

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

MCHC (g/dL)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Male | | | | | | | | | | | | | |
| Mean | 35.2 | 35.7 | 35.1 | 34.8 | 35.5 | 34.7 | | | | | | | |
| Std. Err. | 0.4 | 0.3 | 0.2 | 0.3 | 0.2 | 0.3 | | | | | | | |
| N | 10 | 10 | 10 | 10 | 10 | 10 | | | | | | | |
| .... | | | | | | | | | | | | | |
| Female | | | | | | | | | | | | | |
| Mean | 36.1 | 36.2 | 36.3 | 36.4 | 36.5 | 36.1 | | | | | | | |
| Std. Err. | 0.3 | 0.2 | 0.3 | 0.3 | 0.3 | 0.3 | | | | | | | |
| N | 10 | 9 | 10 | 10 | 9 | 10 | | | | | | | |
| Combined | | | | | | | | | | | | | |
| Mean | 35.7 | 35.9- | 35.7-- | 35.6-- | 35.9-- | 35.4-- | - | - | - | - | - | - | - |
| Std. Err. | 0.3 | 0.2 | 0.2 | 0.3 | 0.2 | 0.3 | | | | | | | |
| N | 20 | 19 | 20 | 20 | 19 | 20 | | | | | | | |

Platelets (x 10 ** 3/UL)

| | | | | | | | | | | | | | |
|---|---|---|---|---|---|---|---|---|---|---|---|---|---|
| Male | | | | | | | | | | | | | |
| Mean | 969 | 1045 | 949 | 1119 | 999 | 1205 | | | | | | | |
| Std. Err. | 68 | 85 | 34 | 63 | 59 | 44 | | | | | | | |
| N | 9 | 10 | 10 | 10 | 10 | 10 | | | | | | | |
| .... | | | | | | | | | | | | | |
| Female | | | | | | | | | | | | | |
| Mean | 935 | 878 | 972 | 1082 | 836 | 934 | | | | | | | |
| Std. Err. | 59 | 79 | 67 | 79 | 68 | 60 | | | | | | | |
| N | 10 | 9 | 10 | 10 | 9 | 9 | | | | | | | |
| Combined | | | | | | | | | | | | | |
| Mean | 951 | 966- | 961-- | 1101a# | 922-- | 1077-- | - | - | - | - | - | - | - |
| Std. Err. | 44 | 60 | 37 | 49 | 47 | 48 | | | | | | | |
| N | 19 | 19 | 20 | 20 | 19 | 19 | | | | | | | |

a Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 4: Blood Assays

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Lymphocytes (%)** | | | | | | | | | | | | | |
| Male | | | | | | | | | | | | | |
| Mean | 62 | 62 | 65 | 63 | 66 | 58 | | | | | | | |
| Std. Err. | 4 | 3 | 5 | 2 | 2 | 4 | | | | | | | |
| N | 10 | 10 | 10 | 10 | 10 | 10 | | | | | | | |
| Female | | | | | | | | | | | | | |
| Mean | 77 | 72 | 76 | 72 | 74 | 76 | | | | | | | |
| Std. Err. | 1 | 2 | 2 | 2 | 3 | 2 | | | | | | | |
| N | 10 | 9 | 10 | 10 | 9 | 10 | | | | | | | |
| Combined | | | | | | | | | | | | | |
| Mean | 69 | 67- | 70-- | 67-- | 70-- | 67-- | - | - | - | - | - | - | - |
| Std. Err. | 3 | 2 | 3 | 2 | 2 | 3 | | | | | | | |
| N | 20 | 19 | 20 | 20 | 19 | 20 | | | | | | | |
| **Neutrophils (%)** | | | | | | | | | | | | | |
| Male | | | | | | | | | | | | | |
| Mean | 35 | 34 | 32 | 32 | 31 | 38 | | | | | | | |
| Std. Err. | 4 | 3 | 5 | 2 | 2 | 5 | | | | | | | |
| N | 10 | 10 | 10 | 10 | 10 | 10 | | | | | | | |
| Female | | | | | | | | | | | | | |
| Mean | 20 | 23 | 19 | 24 | 24 | 20 | | | | | | | |
| Std. Err. | 2 | 3 | 2 | 2 | 3 | 2 | | | | | | | |
| N | 10 | 9 | 10 | 10 | 9 | 10 | | | | | | | |
| Combined | | | | | | | | | | | | | |
| Mean | 28 | 29- | 25-- | 28-- | 27-- | 29-- | - | - | - | - | - | - | - |
| Std. Err. | 3 | 2 | 3 | 2 | 2 | 3 | | | | | | | |
| N | 20 | 19 | 20 | 20 | 19 | 20 | | | | | | | |

a Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 4: Blood Assays
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

**Eosinophils (%)**

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Male | | | | | | | | | | | | | |
| Mean | 2 | 3 | 3 | 3 | 2 | 3 | | | | | | | |
| Std. Err. | 1 | 1 | 1 | 1 | 0 | 1 | | | | | | | |
| N | 10 | 10 | 10 | 10 | 10 | 10 | | | | | | | |
| .... | | | | | | | | | | | | | |
| Female | | | | | | | | | | | | | |
| Mean | 2 | 3 | 4 | 4 | 2 | 4 | | | | | | | |
| Std. Err. | 0 | 1 | 0 | 1 | 0 | 1 | | | | | | | |
| N | 10 | 9 | 10 | 10 | 9 | 10 | | | | | | | |
| Combined | | | | | | | | | | | | | |
| Mean | 2 | 3- | 3-- | 3-- | 2-- | 3-- | - | - | - | - | - | - | - |
| Std. Err. | 0 | 0 | 0 | 0 | 0 | 1 | | | | | | | |
| N | 20 | 19 | 20 | 20 | 19 | 20 | | | | | | | |

**Monocytes (%)**

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Male | | | | | | | | | | | | | |
| Mean | 1 | 1 | 1 | 2 | 1 | 2 | | | | | | | |
| Std. Err. | 0 | 0 | 0 | 1 | 0 | 0 | | | | | | | |
| N | 10 | 10 | 10 | 10 | 10 | 10 | | | | | | | |
| .... | | | | | | | | | | | | | |
| Female | | | | | | | | | | | | | |
| Mean | 1 | 1 | 1 | 1 | 1 | 1 | | | | | | | |
| Std. Err. | 0 | 0 | 0 | 0 | 0 | 0 | | | | | | | |
| N | 10 | 9 | 10 | 10 | 9 | 10 | | | | | | | |
| Combined | | | | | | | | | | | | | |
| Mean | 1 | 1- | 1-- | 1-- | 1-- | 1-- | - | - | - | - | - | - | - |
| Std. Err. | 0 | 0 | 0 | 0 | 0 | 0 | | | | | | | |
| N | 20 | 19 | 20 | 20 | 19 | 20 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 4: Blood Assays
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

**Basophils (%)**

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Male | | | | | | | | | | | | | |
| Mean | 0 | 0 | 0 | 0 | 0 | 0 | | | | | | | |
| Std. Err. | 0 | 0 | 0 | 0 | 0 | 0 | | | | | | | |
| N | 10 | 10 | 10 | 10 | 10 | 10 | | | | | | | |
| .... | | | | | | | | | | | | | |
| Female | | | | | | | | | | | | | |
| Mean | 0 | 0 | 0 | 0 | 0 | 0 | | | | | | | |
| Std. Err. | 0 | 0 | 0 | 0 | 0 | 0 | | | | | | | |
| N | 10 | 9 | 10 | 10 | 9 | 10 | | | | | | | |
| Combined | | | | | | | | | | | | | |
| Mean | 0 | 0- | 0-- | 0-- | 0-- | 0-- | - | - | - | - | - | - | - |
| Std. Err. | 0 | 0 | 0 | 0 | 0 | 0 | | | | | | | |
| N | 20 | 19 | 20 | 20 | 19 | 20 | | | | | | | |

**Bands (%)**

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Male | | | | | | | | | | | | | |
| Mean | 0 | 0 | 0 | 0 | 0 | 0 | | | | | | | |
| Std. Err. | 0 | 0 | 0 | 0 | 0 | 0 | | | | | | | |
| N | 10 | 10 | 10 | 10 | 10 | 10 | | | | | | | |
| .... | | | | | | | | | | | | | |
| Female | | | | | | | | | | | | | |
| Mean | 0 | 0 | 0 | 0 | 0 | 0 | | | | | | | |
| Std. Err. | 0 | 0 | 0 | 0 | 0 | 0 | | | | | | | |
| N | 10 | 9 | 10 | 10 | 9 | 10 | | | | | | | |
| Combined | | | | | | | | | | | | | |
| Mean | 0 | 0- | 0-- | 0-- | 0-- | 0-- | - | - | - | - | - | - | - |
| Std. Err. | 0 | 0 | 0 | 0 | 0 | 0 | | | | | | | |
| N | 20 | 19 | 20 | 20 | 19 | 20 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 4: Blood Assays
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **NRBC (/100 WBC)** | | | | | | | | | | | | | |
| Male | | | | | | | | | | | | | |
| Mean | 0 | 0 | 0 | 0 | 0 | 0 | | | | | | | |
| Std. Err. | 0 | 0 | 0 | 0 | 0 | 0 | | | | | | | |
| N | 10 | 10 | 10 | 10 | 10 | 10 | | | | | | | |
| Female | | | | | | | | | | | | | |
| Mean | 0 | 0 | 0 | 0 | 0 | 0 | | | | | | | |
| Std. Err. | 0 | 0 | 0 | 0 | 0 | 0 | | | | | | | |
| N | 10 | 9 | 10 | 10 | 9 | 10 | | | | | | | |
| Combined | | | | | | | | | | | | | |
| Mean | 0 | 0- | 0-- | 0-- | 0-- | 0-- | - | - | - | - | - | - | - |
| Std. Err. | 0 | 0 | 0 | 0 | 0 | 0 | | | | | | | |
| N | 20 | 19 | 20 | 20 | 19 | 20 | | | | | | | |
| **Aniso (coded)** | | | | | | | | | | | | | |
| Male | | | | | | | | | | | | | |
| Mean | 0.2 | 0.1 | 0.3 | 0.7 | 0.1 | 0.5 | | | | | | | |
| Std. Err. | 0.1 | 0.1 | 0.1 | 0.1 | 0.1 | 0.1 | | | | | | | |
| N | 10 | 10 | 10 | 10 | 10 | 10 | | | | | | | |
| Female | | | | | | | | | | | | | |
| Mean | 0.1 | 0.1 | 0.2 | 0.7 | 0.1 | 0.5 | | | | | | | |
| Std. Err. | 0.1 | 0.1 | 0.1 | 0.1 | 0.1 | 0.1 | | | | | | | |
| N | 10 | 9 | 10 | 10 | 9 | 10 | | | | | | | |
| Combined | | | | | | | | | | | | | |
| Mean | 0.1 | 0.1- | 0.2-- | 0.7a# | 0.1-- | 0.5a# | I | I | - | - | - | - | - |
| Std. Err. | 0.0 | 0.0 | 0.1 | 0.1 | 0.0 | 0.1 | | | | | | | |
| N | 20 | 19 | 20 | 20 | 19 | 20 | | | | | | | |

a Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 4: Blood Assays

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

**Micro (coded)**

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT DR at % Procyst.<br>0.0 | <br>1.0 | <br>DIF | Procysteine DR at mg/kg AZT<br>0 | <br>50 | <br>500 | <br>DIF |
|---|---|---|---|---|---|---|---|---|---|---|---|---|---|
| Male | | | | | | | | | | | | | |
| Mean | 0.0 | 0.0 | 0.1 | 0.0 | 0.0 | 0.1 | | | | | | | |
| Std. Err. | 0.0 | 0.0 | 0.1 | 0.0 | 0.0 | 0.1 | | | | | | | |
| N | 10 | 10 | 10 | 10 | 10 | 10 | | | | | | | |
| .... | | | | | | | | | | | | | |
| Female | | | | | | | | | | | | | |
| Mean | 0.0 | 0.0 | 0.0 | 0.0 | 0.0 | 0.1 | | | | | | | |
| Std. Err. | 0.0 | 0.0 | 0.0 | 0.0 | 0.0 | 0.1 | | | | | | | |
| N | 10 | 9 | 10 | 10 | 9 | 10 | | | | | | | |
| Combined | | | | | | | | | | | | | |
| Mean | 0.0 | 0.0- | 0.0-- | 0.0-- | 0.0-- | 0.1-- | - | - | - | - | - | - | - |
| Std. Err. | 0.0 | 0.0 | 0.0 | 0.0 | 0.0 | 0.0 | | | | | | | |
| N | 20 | 19 | 20 | 20 | 19 | 20 | | | | | | | |

**Macro (coded)**

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT DR at % Procyst.<br>0.0 | <br>1.0 | <br>DIF | Procysteine DR at mg/kg AZT<br>0 | <br>50 | <br>500 | <br>DIF |
|---|---|---|---|---|---|---|---|---|---|---|---|---|---|
| Male | | | | | | | | | | | | | |
| Mean | 0.0 | 0.0 | 0.0 | 0.2 | 0.0 | 0.2 | | | | | | | |
| Std. Err. | 0.0 | 0.0 | 0.0 | 0.1 | 0.0 | 0.1 | | | | | | | |
| N | 10 | 10 | 10 | 10 | 10 | 10 | | | | | | | |
| .... | | | | | | | | | | | | | |
| Female | | | | | | | | | | | | | |
| Mean | 0.0 | 0.0 | 0.0 | 0.1 | 0.0 | 0.1 | | | | | | | |
| Std. Err. | 0.0 | 0.0 | 0.0 | 0.1 | 0.0 | 0.1 | | | | | | | |
| N | 10 | 9 | 10 | 10 | 9 | 10 | | | | | | | |
| Combined | | | | | | | | | | | | | |
| Mean | 0.0 | 0.0- | 0.0-- | 0.2@# | 0.0-- | 0.1-- | - | - | - | - | - | - | - |
| Std. Err. | 0.0 | 0.0 | 0.0 | 0.1 | 0.0 | 0.0 | | | | | | | |
| N | 20 | 19 | 20 | 20 | 19 | 20 | | | | | | | |

@ Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 4: Blood Assays

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT | 0 mg/kg | 0 mg/kg | 50 mg/kg | 500 mg/kg | 50 mg/kg | 500 mg/kg | AZT DR at % Procyst. | | | Procysteine DR at mg/kg AZT | | | |
|---|---|---|---|---|---|---|---|---|---|---|---|---|---|
| Procysteine | 0.0% | 1.0% | 0.0% | 0.0% | 1.0% | 1.0% | 0.0 | 1.0 | DIF | 0 | 50 | 500 | DIF |
| **Poly (coded)** | | | | | | | | | | | | | |
| Male | | | | | | | | | | | | | |
| Mean | 0.7 | 0.8 | 0.9 | 1.0 | 0.7 | 0.8 | | | | | | | |
| Std. Err. | 0.1 | 0.1 | 0.1 | 0.1 | 0.1 | 0.1 | | | | | | | |
| N | 10 | 10 | 10 | 10 | 10 | 10 | | | | | | | |
| .... | | | | | | | | | | | | | |
| Female | | | | | | | | | | | | | |
| Mean | 0.6 | 0.6 | 0.7 | 0.8 | 0.8 | 0.8 | | | | | | | |
| Std. Err. | 0.1 | 0.1 | 0.1 | 0.1 | 0.2 | 0.1 | | | | | | | |
| N | 10 | 9 | 10 | 10 | 9 | 10 | | | | | | | |
| Combined | | | | | | | | | | | | | |
| Mean | 0.6 | 0.7- | 0.8-- | 0.9@- | 0.8-- | 0.8@- | I | - | - | - | - | - | - |
| Std. Err. | 0.0 | 0.1 | 0.1 | 0.0 | 0.1 | 0.1 | | | | | | | |
| N | 20 | 19 | 20 | 20 | 19 | 20 | | | | | | | |
| **Burr Cells (coded)** | | | | | | | | | | | | | |
| Male | | | | | | | | | | | | | |
| Mean | 0.1 | 0.2 | 0.1 | 0.0 | 0.0 | 0.0 | | | | | | | |
| Std. Err. | 0.1 | 0.1 | 0.1 | 0.0 | 0.0 | 0.0 | | | | | | | |
| N | 10 | 10 | 10 | 10 | 10 | 10 | | | | | | | |
| .... | | | | | | | | | | | | | |
| Female | | | | | | | | | | | | | |
| Mean | 0.1 | 0.0 | 0.1 | 0.1 | 0.1 | 0.2 | | | | | | | |
| Std. Err. | 0.1 | 0.0 | 0.1 | 0.1 | 0.1 | 0.1 | | | | | | | |
| N | 10 | 9 | 10 | 10 | 9 | 10 | | | | | | | |
| Combined | | | | | | | | | | | | | |
| Mean | 0.1 | 0.1- | 0.1-- | 0.0-- | 0.1-- | 0.1-- | - | - | - | - | - | - | - |
| Std. Err. | 0.1 | 0.0 | 0.0 | 0.0 | 0.0 | 0.0 | | | | | | | |
| N | 20 | 19 | 20 | 20 | 19 | 20 | | | | | | | |

@ Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 4: Blood Assays

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT DR at % Procyst.<br>0.0 | <br>1.0 | <br>DIF | Procysteine DR at mg/kg AZT<br>0 | <br>50 | <br>500 | <br>DIF |
|---|---|---|---|---|---|---|---|---|---|---|---|---|---|
| **Howell Jolly Bodies (coded)** | | | | | | | | | | | | | |
| Male | | | | | | | | | | | | | |
| Mean | 0.1 | 0.1 | 0.1 | 0.5 | 0.1 | 0.3 | | | | | | | |
| Std. Err. | 0.1 | 0.1 | 0.1 | 0.1 | 0.1 | 0.1 | | | | | | | |
| N | 10 | 10 | 10 | 10 | 10 | 10 | | | | | | | |
| Female | | | | | | | | | | | | | |
| Mean | 0.0 | 0.0 | 0.0 | 0.3 | 0.1 | 0.2 | | | | | | | |
| Std. Err. | 0.0 | 0.0 | 0.0 | 0.1 | 0.1 | 0.1 | | | | | | | |
| N | 10 | 9 | 10 | 10 | 9 | 10 | | | | | | | |
| Combined | | | | | | | | | | | | | |
| Mean | 0.1 | 0.0- | 0.0-- | 0.4@# | 0.1-- | 0.2@# | - | I | - | - | - | - | - |
| Std. Err. | 0.0 | 0.0 | 0.0 | 0.1 | 0.0 | 0.1 | | | | | | | |
| N | 20 | 19 | 20 | 20 | 19 | 20 | | | | | | | |
| **Reticulocytes (%)** | | | | | | | | | | | | | |
| Male | | | | | | | | | | | | | |
| Mean | 1.4 | 1.5 | 1.5 | 1.7 | 1.4 | 1.6 | | | | | | | |
| Std. Err. | 0.1 | 0.2 | 0.1 | 0.1 | 0.2 | 0.1 | | | | | | | |
| N | 10 | 10 | 10 | 10 | 10 | 10 | | | | | | | |
| Female | | | | | | | | | | | | | |
| Mean | 1.3 | 1.4 | 1.4 | 1.3 | 1.5 | 1.3 | | | | | | | |
| Std. Err. | 0.1 | 0.1 | 0.2 | 0.2 | 0.2 | 0.2 | | | | | | | |
| N | 10 | 9 | 10 | 10 | 9 | 9 | | | | | | | |
| Combined | | | | | | | | | | | | | |
| Mean | 1.4 | 1.5- | 1.4-- | 1.5-- | 1.4-- | 1.4-- | - | - | - | - | - | - | - |
| Std. Err. | 0.1 | 0.1 | 0.1 | 0.1 | 0.1 | 0.1 | | | | | | | |
| N | 20 | 19 | 20 | 20 | 19 | 19 | | | | | | | |

@ Indicates Difference From Group 1 : 0 mg/kg AZT, 0.0% Procysteine

\# Indicates Difference From Group 2 : 0 mg/kg AZT, 1.0% Procysteine

\- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 5: Bone Marrow-Myeloid Series %

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT | 0 mg/kg | 0 mg/kg | 50 mg/kg | 500 mg/kg | 50 mg/kg | 500 mg/kg | AZT DR at % Procyst. | | | Procysteine DR at mg/kg AZT | | | |
|---|---|---|---|---|---|---|---|---|---|---|---|---|---|
| Procysteine | 0.0% | 1.0% | 0.0% | 0.0% | 1.0% | 1.0% | 0.0 | 1.0 | DIF | 0 | 50 | 500 | DIF |
| **Myeloblast (%)** | | | | | | | | | | | | | |
| Male | | | | | | | | | | | | | |
| Mean | 3.0 | 3.3- | 3.8a# | 3.5a- | 3.5a- | 3.6a- | | | | - | - | - | - |
| Std. Err. | 0.2 | 0.2 | 0.1 | 0.1 | 0.2 | 0.1 | | | | | | | |
| N | 10 | 10 | 10 | 10 | 10 | 10 | | | | | | | |
| Female | | | | | | | | | | | | | |
| Mean | 2.4 | 3.2a | 3.5a- | 3.6a- | 2.7-- | 3.1a- | | | | I | D | D | * |
| Std. Err. | 0.1 | 0.1 | 0.1 | 0.1 | 0.2 | 0.1 | | | | | | | |
| N | 10 | 8 | 10 | 10 | 8 | 9 | | | | | | | |
| Combined | | | | | | | | | | | | | |
| Mean | 2.7 | 3.2 | 3.6 | 3.6 | 3.1 | 3.3 | I | - | * | | | | |
| Std. Err. | 0.1 | 0.1 | 0.1 | 0.1 | 0.2 | 0.1 | | | | | | | |
| N | 20 | 18 | 20 | 20 | 18 | 19 | | | | | | | |
| **Promyelocyte (%)** | | | | | | | | | | | | | |
| Male | | | | | | | | | | | | | |
| Mean | 9.3 | 8.5 | 13.0 | 12.7 | 9.6 | 9.9 | | | | | | | |
| Std. Err. | 0.2 | 0.2 | 0.2 | 0.3 | 0.1 | 0.1 | | | | | | | |
| N | 10 | 10 | 10 | 10 | 10 | 10 | | | | | | | |
| Female | | | | | | | | | | | | | |
| Mean | 9.0 | 8.4 | 12.1 | 12.5 | 8.9 | 9.1 | | | | | | | |
| Std. Err. | 0.2 | 0.3 | 0.1 | 0.1 | 0.3 | 0.2 | | | | | | | |
| N | 10 | 8 | 10 | 10 | 8 | 9 | | | | | | | |
| Combined | | | | | | | | | | | | | |
| Mean | 9.1 | 8.4a | 12.6a# | 12.6a# | 9.3-# | 9.5-# | I | I | * | D | D | D | * |
| Std. Err. | 0.2 | 0.2 | 0.2 | 0.2 | 0.2 | 0.1 | | | | | | | |
| N | 20 | 18 | 20 | 20 | 18 | 19 | | | | | | | |

a Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 5: Bone Marrow-Myeloid Series %

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Myelocyte (%)** | | | | | | | | | | | | | |
| Male | | | | | | | | | | | | | |
| Mean | 9.1 | 9.6 | 12.9 | 12.4 | 9.6 | 10.0 | | | | | | | |
| Std. Err. | 0.3 | 0.3 | 0.2 | 0.3 | 0.2 | 0.3 | | | | | | | |
| N | 10 | 10 | 10 | 10 | 10 | 10 | | | | | | | |
| Female | | | | | | | | | | | | | |
| Mean | 8.8 | 9.4 | 12.5 | 12.7 | 9.6 | 9.8 | | | | | | | |
| Std. Err. | 0.2 | 0.2 | 0.3 | 0.2 | 0.2 | 0.3 | | | | | | | |
| N | 10 | 8 | 10 | 10 | 8 | 9 | | | | | | | |
| Combined | | | | | | | | | | | | | |
| Mean | 9.0 | 9.5- | 12.7@# | 12.6@# | 9.6-- | 9.9@- | I | - | * | - | D | D | * |
| Std. Err. | 0.2 | 0.2 | 0.2 | 0.2 | 0.1 | 0.2 | | | | | | | |
| N | 20 | 18 | 20 | 20 | 18 | 19 | | | | | | | |
| **Total Proliferating (%)** | | | | | | | | | | | | | |
| Male | | | | | | | | | | | | | |
| Mean | 21.3 | 21.4- | 29.8@# | 28.7@# | 22.7@# | 23.5@# | | | | | | | |
| Std. Err. | 0.3 | 0.2 | 0.3 | 0.4 | 0.3 | 0.3 | | | | | | | |
| N | 10 | 10 | 10 | 10 | 10 | 10 | | | | | | | |
| Female | | | | | | | | | | | | | |
| Mean | 20.2 | 20.9- | 28.0@# | 28.9@# | 21.1-- | 22.0@# | | | | | | | |
| Std. Err. | 0.3 | 0.4 | 0.2 | 0.2 | 0.4 | 0.3 | | | | | | | |
| N | 10 | 8 | 10 | 10 | 8 | 9 | | | | | | | |
| Combined | | | | | | | | | | | | | |
| Mean | 20.7 | 21.2 | 28.9 | 28.8 | 22.0 | 22.8 | I | I | * | - | D | D | * |
| Std. Err. | 0.2 | 0.2 | 0.3 | 0.2 | 0.3 | 0.3 | | | | | | | |
| N | 20 | 18 | 20 | 20 | 18 | 19 | | | | | | | |

@ Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 5: Bone Marrow-Myeloid Series %

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Metamyelocyte (%)** | | | | | | | | | | | | | |
| Male | | | | | | | | | | | | | |
| Mean | 8.8 | 8.0- | 8.7-- | 8.6-- | 9.8@# | 9.0-# | | | | | | | |
| Std. Err. | 0.3 | 0.3 | 0.2 | 0.2 | 0.3 | 0.2 | | | | | | | |
| N | 10 | 10 | 10 | 10 | 10 | 10 | | | | | | | |
| Female | | | | | | | | | | | | | |
| Mean | 8.4 | 8.5- | 8.9-- | 9.2-- | 8.8-- | 9.9@# | | | | | | | |
| Std. Err. | 0.1 | 0.4 | 0.2 | 0.2 | 0.4 | 0.2 | | | | | | | |
| N | 10 | 8 | 10 | 10 | 8 | 9 | | | | | | | |
| Combined | | | | | | | | | | | | | |
| Mean | 8.6 | 8.2 | 8.8 | 8.9 | 9.3 | 9.4 | - | I | * | - | - | - | * |
| Std. Err. | 0.1 | 0.2 | 0.1 | 0.2 | 0.2 | 0.2 | | | | | | | |
| N | 20 | 18 | 20 | 20 | 18 | 19 | | | | | | | |
| **Band (%)** | | | | | | | | | | | | | |
| Male | | | | | | | | | | | | | |
| Mean | 15.2 | 15.8- | 13.6@# | 14.0@# | 15.2-- | 15.5-- | | | | | | | |
| Std. Err. | 0.3 | 0.2 | 0.2 | 0.2 | 0.1 | 0.2 | | | | | | | |
| N | 10 | 10 | 10 | 10 | 10 | 10 | | | | | | | |
| Female | | | | | | | | | | | | | |
| Mean | 15.6 | 15.5- | 14.9-- | 13.7@# | 15.3-- | 15.7-- | | | | | | | |
| Std. Err. | 0.1 | 0.3 | 0.2 | 0.3 | 0.3 | 0.3 | | | | | | | |
| N | 10 | 8 | 10 | 10 | 8 | 9 | | | | | | | |
| Combined | | | | | | | | | | | | | |
| Mean | 15.4 | 15.6 | 14.2 | 13.8 | 15.3 | 15.6 | D | - | * | - | I | I | * |
| Std. Err. | 0.2 | 0.2 | 0.2 | 0.2 | 0.1 | 0.1 | | | | | | | |
| N | 20 | 18 | 20 | 20 | 18 | 19 | | | | | | | |

@ Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 5: Bone Marrow-Myeloid Series %

Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

**Neutrophil (%)**

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Male | | | | | | | | | | | | | |
| Mean | 13.4 | 13.4- | 12.3@# | 12.9-- | 12.9-- | 13.5-- | | | | | | | |
| Std. Err. | 0.3 | 0.3 | 0.2 | 0.1 | 0.2 | 0.2 | | | | | | | |
| N | 10 | 10 | 10 | 10 | 10 | 10 | | | | | | | |
| Female | | | | | | | | | | | | | |
| Mean | 13.2 | 13.7- | 12.5-# | 12.4@# | 13.6-- | 12.4-# | | | | | | | |
| Std. Err. | 0.1 | 0.3 | 0.2 | 0.3 | 0.3 | 0.2 | | | | | | | |
| N | 10 | 8 | 10 | 10 | 8 | 9 | | | | | | | |
| Combined | | | | | | | | | | | | | |
| Mean | 13.3 | 13.5 | 12.4 | 12.6 | 13.2 | 13.0 | D | - | - | - | I | - | - |
| Std. Err. | 0.2 | 0.2 | 0.1 | 0.2 | 0.2 | 0.2 | | | | | | | |
| N | 20 | 18 | 20 | 20 | 18 | 19 | | | | | | | |

**Eosinophil (%)**

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Male | | | | | | | | | | | | | |
| Mean | 3.7 | 4.4- | 2.8@# | 2.9@# | 2.9@# | 3.5-# | | | | | | | |
| Std. Err. | 0.2 | 0.2 | 0.1 | 0.2 | 0.2 | 0.2 | | | | | | | |
| N | 10 | 10 | 10 | 10 | 10 | 10 | | | | | | | |
| Female | | | | | | | | | | | | | |
| Mean | 3.4 | 4.3@ | 3.0-# | 2.2@# | 4.2@- | 3.2-# | | | | | | | |
| Std. Err. | 0.2 | 0.1 | 0.2 | 0.1 | 0.2 | 0.3 | | | | | | | |
| N | 10 | 8 | 10 | 10 | 8 | 9 | | | | | | | |
| Combined | | | | | | | | | | | | | |
| Mean | 3.5 | 4.4 | 2.9 | 2.6 | 3.5 | 3.3 | D | D | - | I | I | I | - |
| Std. Err. | 0.2 | 0.1 | 0.1 | 0.1 | 0.2 | 0.2 | | | | | | | |
| N | 20 | 18 | 20 | 20 | 18 | 19 | | | | | | | |

@ Indicates Difference From Group 1 : 0 mg/kg AZT, 0.0% Procysteine
\# Indicates Difference From Group 2 : 0 mg/kg AZT, 1.0% Procysteine
\- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 5: Bone Marrow-Myeloid Series %
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | AZT DR at % Procyst. 1.0 | AZT DR at % Procyst. DIF | Procysteine DR at mg/kg AZT 0 | Procysteine DR at mg/kg AZT 50 | Procysteine DR at mg/kg AZT 500 | Procysteine DR at mg/kg AZT DIF |
|---|---|---|---|---|---|---|---|---|---|---|---|---|---|
| **Basophil (%)** | | | | | | | | | | | | | |
| Male | | | | | | | | | | | | | |
| Mean | 0.0 | 0.0 | 0.0 | 0.0 | 0.0 | 0.0 | | | | | | | |
| Std. Err. | 0.0 | 0.0 | 0.0 | 0.0 | 0.0 | 0.0 | | | | | | | |
| N | 10 | 10 | 10 | 10 | 10 | 10 | | | | | | | |
| Female | | | | | | | | | | | | | |
| Mean | 0.0 | 0.0 | 0.0 | 0.0 | 0.0 | 0.0 | | | | | | | |
| Std. Err. | 0.0 | 0.0 | 0.0 | 0.0 | 0.0 | 0.0 | | | | | | | |
| N | 10 | 8 | 10 | 10 | 8 | 9 | | | | | | | |
| Combined | | | | | | | - | - | - | - | - | - | - |
| Mean | 0.0 | 0.0- | 0.0-- | 0.0-- | 0.0-- | 0.0-- | | | | | | | |
| Std. Err. | 0.0 | 0.0 | 0.0 | 0.0 | 0.0 | 0.0 | | | | | | | |
| N | 20 | 18 | 20 | 20 | 18 | 19 | | | | | | | |
| **Total Myeloid (%)** | | | | | | | | | | | | | |
| Male | | | | | | | | | | | | | |
| Mean | 62.4 | 62.9- | 67.2@# | 67.0@# | 63.6@- | 64.9@# | | | | | | | |
| Std. Err. | 0.3 | 0.2 | 0.2 | 0.3 | 0.3 | 0.2 | | | | | | | |
| N | 10 | 10 | 10 | 10 | 10 | 10 | | | | | | | |
| Female | | | | | | | | | | | | | |
| Mean | 60.8 | 62.8@ | 67.4@# | 66.3@# | 63.1@- | 63.3@- | | | | | | | |
| Std. Err. | 0.4 | 0.3 | 0.3 | 0.2 | 0.3 | 0.3 | | | | | | | |
| N | 10 | 8 | 10 | 10 | 8 | 9 | | | | | | | |
| Combined | | | | | | | I | I | * | I | D | D | * |
| Mean | 61.6 | 62.9 | 67.3 | 66.7 | 63.3 | 64.1 | | | | | | | |
| Std. Err. | 0.3 | 0.2 | 0.2 | 0.2 | 0.2 | 0.2 | | | | | | | |
| N | 20 | 18 | 20 | 20 | 18 | 19 | | | | | | | |

@ Indicates Difference From Group 1 : 0 mg/kg AZT, 0.0% Procysteine
\# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 5a: Bone Marrow-Myeloid Series / Femur x 10**6

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT | 0 mg/kg | 0 mg/kg | 50 mg/kg | 500 mg/kg | 50 mg/kg | 500 mg/kg | AZT DR at % Procyst. | | | Procysteine DR at mg/kg AZT | | | |
|---|---|---|---|---|---|---|---|---|---|---|---|---|---|
| Procysteine | 0.0% | 1.0% | 0.0% | 0.0% | 1.0% | 1.0% | 0.0 | 1.0 | DIF | 0 | 50 | 500 | DIF |
| **Myeloblast (/ Femur x 10**6)** | | | | | | | | | | | | | |
| Male | | | | | | | | | | | | | |
| Mean | 0.35 | 0.42 | 0.44 | 0.37 | 0.45 | 0.36 | | | | | | | |
| Std. Err. | 0.02 | 0.02 | 0.02 | 0.02 | 0.03 | 0.01 | | | | | | | |
| N | 10 | 10 | 10 | 10 | 10 | 10 | | | | | | | |
| .... | | | | | | | | | | | | | |
| Female | | | | | | | | | | | | | |
| Mean | 0.27 | 0.38 | 0.35 | 0.32 | 0.27 | 0.28 | | | | | | | |
| Std. Err. | 0.02 | 0.02 | 0.03 | 0.01 | 0.01 | 0.02 | | | | | | | |
| N | 10 | 8 | 10 | 10 | 8 | 9 | | | | | | | |
| Combined | | | | | | | | | | | | | |
| Mean | 0.31 | 0.40@ | 0.40@- | 0.34-- | 0.37-- | 0.32-# | - | D | * | I | - | - | * |
| Std. Err. | 0.02 | 0.02 | 0.02 | 0.01 | 0.03 | 0.02 | | | | | | | |
| N | 20 | 18 | 20 | 20 | 18 | 19 | | | | | | | |
| **Promyelocyte (/ Femur x 10**6)** | | | | | | | | | | | | | |
| Male | | | | | | | | | | | | | |
| Mean | 1.10 | 1.08 | 1.53 | 1.34 | 1.21 | 0.99 | | | | | | | |
| Std. Err. | 0.06 | 0.03 | 0.06 | 0.06 | 0.05 | 0.04 | | | | | | | |
| N | 10 | 10 | 10 | 10 | 10 | 10 | | | | | | | |
| .... | | | | | | | | | | | | | |
| Female | | | | | | | | | | | | | |
| Mean | 1.02 | 0.99 | 1.20 | 1.10 | 0.93 | 0.82 | | | | | | | |
| Std. Err. | 0.04 | 0.06 | 0.06 | 0.05 | 0.08 | 0.05 | | | | | | | |
| N | 10 | 8 | 10 | 10 | 8 | 9 | | | | | | | |
| Combined | | | | | | | | | | | | | |
| Mean | 1.06 | 1.04- | 1.36@# | 1.22@# | 1.08-- | 0.91@- | I | - | * | - | D | D | * |
| Std. Err. | 0.04 | 0.03 | 0.06 | 0.05 | 0.06 | 0.04 | | | | | | | |
| N | 20 | 18 | 20 | 20 | 18 | 19 | | | | | | | |

@ Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 5a: Bone Marrow-Myeloid Series / Femur x 10**6

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Myelocyte (/ Femur x 10**6)** | | | | | | | | | | | | | |
| Male | | | | | | | | | | | | | |
| Mean | 1.08 | 1.23 | 1.51 | 1.31 | 1.21 | 1.00 | | | | | | | |
| Std. Err. | 0.08 | 0.06 | 0.06 | 0.07 | 0.06 | 0.05 | | | | | | | |
| N | 10 | 10 | 10 | 10 | 10 | 10 | | | | | | | |
| Female | | | | | | | | | | | | | |
| Mean | 1.01 | 1.11 | 1.24 | 1.12 | 0.99 | 0.88 | | | | | | | |
| Std. Err. | 0.05 | 0.07 | 0.07 | 0.05 | 0.07 | 0.05 | | | | | | | |
| N | 10 | 8 | 10 | 10 | 8 | 9 | | | | | | | |
| Combined | | | | | | | | | | | | | |
| Mean | 1.04 | 1.18- | 1.37a# | 1.21a- | 1.11-- | 0.95-# | I | D | * | - | D | D | * |
| Std. Err. | 0.05 | 0.05 | 0.05 | 0.05 | 0.05 | 0.04 | | | | | | | |
| N | 20 | 18 | 20 | 20 | 18 | 19 | | | | | | | |
| **Total Proliferating (/ Femur x 10**6)** | | | | | | | | | | | | | |
| Male | | | | | | | | | | | | | |
| Mean | 2.53 | 2.73 | 3.48 | 3.02 | 2.87 | 2.35 | | | | | | | |
| Std. Err. | 0.14 | 0.10 | 0.13 | 0.14 | 0.14 | 0.09 | | | | | | | |
| N | 10 | 10 | 10 | 10 | 10 | 10 | | | | | | | |
| Female | | | | | | | | | | | | | |
| Mean | 2.30 | 2.48 | 2.78 | 2.53 | 2.18 | 1.98 | | | | | | | |
| Std. Err. | 0.10 | 0.13 | 0.14 | 0.11 | 0.15 | 0.10 | | | | | | | |
| N | 10 | 8 | 10 | 10 | 8 | 9 | | | | | | | |
| Combined | | | | | | | | | | | | | |
| Mean | 2.41 | 2.62- | 3.13a# | 2.78a- | 2.56-- | 2.18-# | I | D | * | - | D | D | * |
| Std. Err. | 0.09 | 0.08 | 0.12 | 0.10 | 0.13 | 0.08 | | | | | | | |
| N | 20 | 18 | 20 | 20 | 18 | 19 | | | | | | | |

a Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 5a: Bone Marrow-Myeloid Series / Femur x 10**6
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

Metamyelocyte (/ Femur x 10**6)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Male | | | | | | | | | | | | | |
| Mean | 1.04 | 1.01 | 1.02 | 0.90 | 1.23 | 0.90 | | | | | | | |
| Std. Err. | 0.07 | 0.04 | 0.05 | 0.04 | 0.06 | 0.04 | | | | | | | |
| N | 10 | 10 | 10 | 10 | 10 | 10 | | | | | | | |
| .... | | | | | | | | | | | | | |
| Female | | | | | | | | | | | | | |
| Mean | 0.96 | 1.01 | 0.88 | 0.80 | 0.92 | 0.89 | | | | | | | |
| Std. Err. | 0.04 | 0.08 | 0.03 | 0.03 | 0.08 | 0.05 | | | | | | | |
| N | 10 | 8 | 10 | 10 | 8 | 9 | | | | | | | |
| Combined | | | | | | | | | | | | | |
| Mean | 1.00 | 1.01- | 0.95-- | 0.85a# | 1.09-- | 0.90-- | D | - | - | - | - | - | - |
| Std. Err. | 0.04 | 0.04 | 0.03 | 0.03 | 0.06 | 0.03 | | | | | | | |
| N | 20 | 18 | 20 | 20 | 18 | 19 | | | | | | | |

Band (/ Femur x 10**6)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Male | | | | | | | | | | | | | |
| Mean | 1.80 | 2.01 | 1.59 | 1.47 | 1.92 | 1.55 | | | | | | | |
| Std. Err. | 0.10 | 0.06 | 0.06 | 0.06 | 0.08 | 0.05 | | | | | | | |
| N | 10 | 10 | 10 | 10 | 10 | 10 | | | | | | | |
| .... | | | | | | | | | | | | | |
| Female | | | | | | | | | | | | | |
| Mean | 1.78 | 1.84 | 1.48 | 1.20 | 1.58 | 1.41 | | | | | | | |
| Std. Err. | 0.08 | 0.09 | 0.08 | 0.05 | 0.10 | 0.07 | | | | | | | |
| N | 10 | 8 | 10 | 10 | 8 | 9 | | | | | | | |
| Combined | | | | | | | | | | | | | |
| Mean | 1.79 | 1.93- | 1.53a# | 1.33a# | 1.77-- | 1.48a# | D | D | - | - | I | - | - |
| Std. Err. | 0.06 | 0.06 | 0.05 | 0.05 | 0.07 | 0.04 | | | | | | | |
| N | 20 | 18 | 20 | 20 | 18 | 19 | | | | | | | |

a Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 5a: Bone Marrow-Myeloid Series / Femur x 10**6
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

Neutrophil (/ Femur x 10**6)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Male** | | | | | | | | | | | | | |
| Mean | 1.59 | 1.70 | 1.43 | 1.36 | 1.63 | 1.35 | | | | | | | |
| Std. Err. | 0.10 | 0.06 | 0.05 | 0.06 | 0.08 | 0.05 | | | | | | | |
| N | 10 | 10 | 10 | 10 | 10 | 10 | | | | | | | |
| .... | | | | | | | | | | | | | |
| **Female** | | | | | | | | | | | | | |
| Mean | 1.51 | 1.63 | 1.24 | 1.09 | 1.40 | 1.12 | | | | | | | |
| Std. Err. | 0.07 | 0.10 | 0.05 | 0.05 | 0.09 | 0.06 | | | | | | | |
| N | 10 | 8 | 10 | 10 | 8 | 9 | | | | | | | |
| **Combined** | | | | | | | | | | | | | |
| Mean | 1.55 | 1.67- | 1.34a# | 1.22a# | 1.53-- | 1.24a# | D | D | - | - | I | - | - |
| Std. Err. | 0.06 | 0.06 | 0.04 | 0.05 | 0.06 | 0.05 | | | | | | | |
| N | 20 | 18 | 20 | 20 | 18 | 19 | | | | | | | |

Eosinophil (/ Femur x 10**6)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Male** | | | | | | | | | | | | | |
| Mean | 0.43 | 0.57 | 0.33 | 0.31 | 0.37 | 0.35 | | | | | | | |
| Std. Err. | 0.03 | 0.04 | 0.02 | 0.03 | 0.02 | 0.02 | | | | | | | |
| N | 10 | 10 | 10 | 10 | 10 | 10 | | | | | | | |
| .... | | | | | | | | | | | | | |
| **Female** | | | | | | | | | | | | | |
| Mean | 0.39 | 0.51 | 0.29 | 0.19 | 0.44 | 0.28 | | | | | | | |
| Std. Err. | 0.03 | 0.04 | 0.02 | 0.01 | 0.04 | 0.02 | | | | | | | |
| N | 10 | 8 | 10 | 10 | 8 | 9 | | | | | | | |
| **Combined** | | | | | | | | | | | | | |
| Mean | 0.41 | 0.55a | 0.31a# | 0.25a# | 0.40-# | 0.32a# | D | D | - | I | I | - | - |
| Std. Err. | 0.02 | 0.03 | 0.01 | 0.02 | 0.02 | 0.02 | | | | | | | |
| N | 20 | 18 | 20 | 20 | 18 | 19 | | | | | | | |

a Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 5a: Bone Marrow-Myeloid Series / Femur x 10**6
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| AZT | 0 mg/kg | 0 mg/kg | 50 mg/kg | 500 mg/kg | 50 mg/kg | 500 mg/kg | AZT DR at % Procyst. | | | Procysteine DR at mg/kg AZT | | | |
|---|---|---|---|---|---|---|---|---|---|---|---|---|---|
| Procysteine | 0.0% | 1.0% | 0.0% | 0.0% | 1.0% | 1.0% | 0.0 | 1.0 | DIF | 0 | 50 | 500 | DIF |
| **Basophil (/ Femur x 10**6)** | | | | | | | | | | | | | |
| Male | | | | | | | | | | | | | |
| Mean | 0.000 | 0.002 | 0.002 | 0.000 | 0.005 | 0.000 | | | | | | | |
| Std. Err. | 0.000 | 0.002 | 0.002 | 0.000 | 0.004 | 0.000 | | | | | | | |
| N | 10 | 10 | 10 | 10 | 10 | 10 | | | | | | | |
| .... | | | | | | | | | | | | | |
| Female | | | | | | | | | | | | | |
| Mean | 0.004 | 0.000 | 0.002 | 0.003 | 0.003 | 0.002 | | | | | | | |
| Std. Err. | 0.003 | 0.000 | 0.002 | 0.002 | 0.003 | 0.002 | | | | | | | |
| N | 10 | 8 | 10 | 10 | 8 | 9 | | | | | | | |
| Combined | | | | | | | | | | | | | |
| Mean | 0.002 | 0.001- | 0.002-- | 0.002-- | 0.004-- | 0.001-- | - | - | - | - | - | - | - |
| Std. Err. | 0.002 | 0.001 | 0.001 | 0.001 | 0.002 | 0.001 | | | | | | | |
| N | 20 | 18 | 20 | 20 | 18 | 19 | | | | | | | |
| **Total Myeloid (/ Femur x 10**6)** | | | | | | | | | | | | | |
| Male | | | | | | | | | | | | | |
| Mean | 7.39 | 8.03 | 7.85 | 7.05 | 8.02 | 6.50 | | | | | | | |
| Std. Err. | 0.40 | 0.27 | 0.29 | 0.30 | 0.35 | 0.23 | | | | | | | |
| N | 10 | 10 | 10 | 10 | 10 | 10 | | | | | | | |
| .... | | | | | | | | | | | | | |
| Female | | | | | | | | | | | | | |
| Mean | 6.93 | 7.47 | 6.68 | 5.82 | 6.53 | 5.69 | | | | | | | |
| Std. Err. | 0.31 | 0.41 | 0.30 | 0.23 | 0.42 | 0.27 | | | | | | | |
| N | 10 | 8 | 10 | 10 | 8 | 9 | | | | | | | |
| Combined | | | | | | | | | | | | | |
| Mean | 7.16 | 7.78- | 7.27-- | 6.43-# | 7.35-- | 6.12@# | - | D | - | - | - | - | - |
| Std. Err. | 0.25 | 0.24 | 0.24 | 0.23 | 0.32 | 0.20 | | | | | | | |
| N | 20 | 18 | 20 | 20 | 18 | 19 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 6: Bone Marrow-Erythroid Series %

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT | 0 mg/kg | 0 mg/kg | 50 mg/kg | 500 mg/kg | 50 mg/kg | 500 mg/kg | AZT DR at % Procyst. | | | Procysteine DR at mg/kg AZT | | | |
|---|---|---|---|---|---|---|---|---|---|---|---|---|---|
| Procysteine | 0.0% | 1.0% | 0.0% | 0.0% | 1.0% | 1.0% | 0.0 | 1.0 | DIF | 0 | 50 | 500 | DIF |
| **Rubriblast (%)** | | | | | | | | | | | | | |
| Male | | | | | | | | | | | | | |
| Mean | 1.1 | 1.1 | 0.7 | 0.7 | 1.1 | 1.0 | | | | | | | |
| Std. Err. | 0.1 | 0.1 | 0.1 | 0.1 | 0.1 | 0.1 | | | | | | | |
| N | 10 | 10 | 10 | 10 | 10 | 10 | | | | | | | |
| Female | | | | | | | | | | | | | |
| Mean | 1.1 | 1.0 | 0.5 | 0.6 | 1.1 | 1.1 | | | | | | | |
| Std. Err. | 0.1 | 0.1 | 0.0 | 0.0 | 0.1 | 0.1 | | | | | | | |
| N | 10 | 8 | 10 | 10 | 8 | 9 | | | | | | | |
| Combined | | | | | | | | | | | | | |
| Mean | 1.1 | 1.0- | 0.6@# | 0.7@# | 1.1-- | 1.0-- | D | - | * | - | I | I | * |
| Std. Err. | 0.0 | 0.0 | 0.0 | 0.0 | 0.0 | 0.0 | | | | | | | |
| N | 20 | 18 | 20 | 20 | 18 | 19 | | | | | | | |
| **Prorubricyte (%)** | | | | | | | | | | | | | |
| Male | | | | | | | | | | | | | |
| Mean | 8.7 | 8.0- | 9.9@# | 9.4-# | 9.3-# | 9.0-# | | | | | | | |
| Std. Err. | 0.3 | 0.2 | 0.2 | 0.2 | 0.2 | 0.2 | | | | | | | |
| N | 10 | 10 | 10 | 10 | 10 | 10 | | | | | | | |
| Female | | | | | | | | | | | | | |
| Mean | 8.7 | 8.1- | 8.8-- | 10.7@# | 8.1-- | 8.8-- | | | | | | | |
| Std. Err. | 0.3 | 0.3 | 0.2 | 0.3 | 0.2 | 0.4 | | | | | | | |
| N | 10 | 8 | 10 | 10 | 8 | 9 | | | | | | | |
| Combined | | | | | | | | | | | | | |
| Mean | 8.7 | 8.0 | 9.3 | 10.0 | 8.7 | 8.9 | I | I | - | D | - | D | - |
| Std. Err. | 0.2 | 0.2 | 0.2 | 0.2 | 0.2 | 0.2 | | | | | | | |
| N | 20 | 18 | 20 | 20 | 18 | 19 | | | | | | | |

@ Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 6: Bone Marrow-Erythroid Series %

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Rubricyte (%)** | | | | | | | | | | | | | |
| Male | | | | | | | | | | | | | |
| Mean | 22.0 | 22.7- | 19.9@# | 20.2@# | 23.6@- | 22.8-- | D | - | * | - | I | I | * |
| Std. Err. | 0.3 | 0.2 | 0.1 | 0.3 | 0.2 | 0.2 | | | | | | | |
| N | 10 | 10 | 10 | 10 | 10 | 10 | | | | | | | |
| Female | | | | | | | | | | | | | |
| Mean | 23.4 | 22.2@ | 19.9@# | 20.0@# | 22.2@- | 21.6@- | D | - | * | D | I | I | * |
| Std. Err. | 0.2 | 0.4 | 0.2 | 0.1 | 0.3 | 0.3 | | | | | | | |
| N | 10 | 8 | 10 | 10 | 8 | 9 | | | | | | | |
| Combined | | | | | | | | | | | | | |
| Mean | 22.7 | 22.5 | 19.9 | 20.1 | 23.0 | 22.3 | | | | | | | |
| Std. Err. | 0.3 | 0.2 | 0.1 | 0.2 | 0.2 | 0.2 | | | | | | | |
| N | 20 | 18 | 20 | 20 | 18 | 19 | | | | | | | |
| **Total Proliferating (%)** | | | | | | | | | | | | | |
| Male | | | | | | | | | | | | | |
| Mean | 31.8 | 31.7- | 30.5@# | 30.3@# | 34.0@# | 32.8-# | | | | - | I | I | * |
| Std. Err. | 0.3 | 0.4 | 0.1 | 0.3 | 0.4 | 0.2 | | | | | | | |
| N | 10 | 10 | 10 | 10 | 10 | 10 | | | | | | | |
| Female | | | | | | | | | | | | | |
| Mean | 33.3 | 31.3@ | 29.2@# | 31.3@- | 31.3@- | 31.5@- | | | | D | I | - | * |
| Std. Err. | 0.5 | 0.3 | 0.2 | 0.2 | 0.2 | 0.2 | | | | | | | |
| N | 10 | 8 | 10 | 10 | 8 | 9 | | | | | | | |
| Combined | | | | | | | | | | | | | |
| Mean | 32.6 | 31.5 | 29.9 | 30.8 | 32.8 | 32.2 | D | - | * | | | | |
| Std. Err. | 0.3 | 0.2 | 0.2 | 0.2 | 0.4 | 0.2 | | | | | | | |
| N | 20 | 18 | 20 | 20 | 18 | 19 | | | | | | | |

@ Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 6: Bone Marrow-Erythroid Series %

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Metarubricyte (%)** | | | | | | | | | | | | | |
| Male | | | | | | | | | | | | | |
| Mean | 4.5 | 4.0- | 1.0@# | 1.5@# | 1.0@# | 1.0@# | D | D | - | - | - | - | - |
| Std. Err. | 0.2 | 0.2 | 0.1 | 0.1 | 0.1 | 0.1 | | | | | | | |
| N | 10 | 10 | 10 | 10 | 10 | 10 | | | | | | | |
| .... | | | | | | | | | | | | | |
| Female | | | | | | | | | | | | | |
| Mean | 4.3 | 4.2- | 2.2@# | 1.2@# | 4.0-- | 3.8-- | D | - | * | - | I | I | * |
| Std. Err. | 0.3 | 0.2 | 0.2 | 0.1 | 0.2 | 0.2 | | | | | | | |
| N | 10 | 8 | 10 | 10 | 8 | 9 | | | | | | | |
| Combined | | | | | | | | | | | | | |
| Mean | 4.4 | 4.1 | 1.6 | 1.3 | 2.3 | 2.4 | | | | | | | |
| Std. Err. | 0.2 | 0.1 | 0.2 | 0.1 | 0.4 | 0.3 | | | | | | | |
| N | 20 | 18 | 20 | 20 | 18 | 19 | | | | | | | |
| **Total Erythroid (%)** | | | | | | | | | | | | | |
| Male | | | | | | | | | | | | | |
| Mean | 36.3 | 35.7- | 31.5@# | 31.8@# | 35.0@- | 33.9@# | | | | | | | |
| Std. Err. | 0.3 | 0.3 | 0.1 | 0.3 | 0.3 | 0.2 | | | | | | | |
| N | 10 | 10 | 10 | 10 | 10 | 10 | | | | | | | |
| .... | | | | | | | | | | | | | |
| Female | | | | | | | | | | | | | |
| Mean | 37.6 | 35.4@ | 31.4@# | 32.4@# | 35.2@- | 35.4@- | | | | | | | |
| Std. Err. | 0.3 | 0.1 | 0.3 | 0.2 | 0.2 | 0.2 | | | | | | | |
| N | 10 | 8 | 10 | 10 | 8 | 9 | | | | | | | |
| Combined | | | | | | | | | | | | | |
| Mean | 37.0 | 35.6 | 31.5 | 32.1 | 35.1 | 34.6 | D | - | * | D | I | I | * |
| Std. Err. | 0.3 | 0.2 | 0.2 | 0.2 | 0.2 | 0.2 | | | | | | | |
| N | 20 | 18 | 20 | 20 | 18 | 19 | | | | | | | |

@ Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 6a: Bone Marrow-Erythroid Series / Femur x 10**6

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Rubriblast (/ Femur x 10**6)** | | | | | | | | | | | | | |
| Male | | | | | | | | | | | | | |
| Mean | 0.13 | 0.14 | 0.08 | 0.08 | 0.14 | 0.10 | | | | | | | |
| Std. Err. | 0.01 | 0.01 | 0.01 | 0.01 | 0.01 | 0.01 | | | | | | | |
| N | 10 | 10 | 10 | 10 | 10 | 10 | | | | | | | |
| Female | | | | | | | | | | | | | |
| Mean | 0.13 | 0.12 | 0.05 | 0.05 | 0.11 | 0.10 | | | | | | | |
| Std. Err. | 0.01 | 0.01 | 0.00 | 0.00 | 0.01 | 0.01 | | | | | | | |
| N | 10 | 8 | 10 | 10 | 8 | 9 | | | | | | | |
| Combined | | | | | | | | | | | | | |
| Mean | 0.13 | 0.13- | 0.07a# | 0.06a# | 0.12-- | 0.10a# | D | D | * | - | I | I | * |
| Std. Err. | 0.01 | 0.01 | 0.01 | 0.01 | 0.01 | 0.00 | | | | | | | |
| N | 20 | 18 | 20 | 20 | 18 | 19 | | | | | | | |
| **Prorubricyte (/ Femur x 10**6)** | | | | | | | | | | | | | |
| Male | | | | | | | | | | | | | |
| Mean | 1.03 | 1.01- | 1.16-- | 0.99-- | 1.17-- | 0.91-- | | | | | | | |
| Std. Err. | 0.06 | 0.03 | 0.04 | 0.05 | 0.07 | 0.04 | | | | | | | |
| N | 10 | 10 | 10 | 10 | 10 | 10 | | | | | | | |
| Female | | | | | | | | | | | | | |
| Mean | 0.99 | 0.96- | 0.87-- | 0.94-- | 0.84-- | 0.80-- | | | | | | | |
| Std. Err. | 0.05 | 0.07 | 0.05 | 0.04 | 0.06 | 0.06 | | | | | | | |
| N | 10 | 8 | 10 | 10 | 8 | 9 | | | | | | | |
| Combined | | | | | | | | | | | | | |
| Mean | 1.01 | 0.99 | 1.01 | 0.96 | 1.02 | 0.86 | - | - | - | - | - | - | - |
| Std. Err. | 0.04 | 0.04 | 0.05 | 0.03 | 0.06 | 0.04 | | | | | | | |
| N | 20 | 18 | 20 | 20 | 18 | 19 | | | | | | | |

a Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 6a: Bone Marrow-Erythroid Series / Femur x 10**6

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT | 0 mg/kg | 0 mg/kg | 50 mg/kg | 500 mg/kg | 50 mg/kg | 500 mg/kg | AZT DR at % Procyst. 0.0 | AZT DR at % Procyst. 1.0 | AZT DR at % Procyst. DIF | Procysteine DR at mg/kg AZT 0 | Procysteine DR at mg/kg AZT 50 | Procysteine DR at mg/kg AZT 500 | Procysteine DR at mg/kg AZT DIF |
|---|---|---|---|---|---|---|---|---|---|---|---|---|---|
| Procysteine | 0.0% | 1.0% | 0.0% | 0.0% | 1.0% | 1.0% | | | | | | | |
| **Rubricyte (/ Femur x 10**6)** | | | | | | | | | | | | | |
| Male | | | | | | | | | | | | | |
| Mean | 2.61 | 2.90 | 2.33 | 2.12 | 2.98 | 2.28 | | | | | | | |
| Std. Err. | 0.15 | 0.11 | 0.09 | 0.08 | 0.13 | 0.07 | | | | | | | |
| N | 10 | 10 | 10 | 10 | 10 | 10 | | | | | | | |
| .... | | | | | | | | | | | | | |
| Female | | | | | | | | | | | | | |
| Mean | 2.67 | 2.63 | 1.98 | 1.75 | 2.29 | 1.94 | | | | | | | |
| Std. Err. | 0.13 | 0.12 | 0.09 | 0.08 | 0.14 | 0.09 | | | | | | | |
| N | 10 | 8 | 10 | 10 | 8 | 9 | | | | | | | |
| Combined | | | | | | | | | | | | | |
| Mean | 2.64 | 2.78- | 2.15@# | 1.94@# | 2.67-- | 2.12@# | D | D | - | - | I | - | - |
| Std. Err. | 0.09 | 0.09 | 0.08 | 0.07 | 0.13 | 0.07 | | | | | | | |
| N | 20 | 18 | 20 | 20 | 18 | 19 | | | | | | | |
| **Total Proliferating (/ Femur x 10**6)** | | | | | | | | | | | | | |
| Male | | | | | | | | | | | | | |
| Mean | 3.77 | 4.04 | 3.57 | 3.18 | 4.29 | 3.29 | | | | | | | |
| Std. Err. | 0.21 | 0.14 | 0.14 | 0.12 | 0.20 | 0.11 | | | | | | | |
| N | 10 | 10 | 10 | 10 | 10 | 10 | | | | | | | |
| .... | | | | | | | | | | | | | |
| Female | | | | | | | | | | | | | |
| Mean | 3.79 | 3.71 | 2.90 | 2.74 | 3.24 | 2.84 | | | | | | | |
| Std. Err. | 0.18 | 0.18 | 0.14 | 0.11 | 0.21 | 0.15 | | | | | | | |
| N | 10 | 8 | 10 | 10 | 8 | 9 | | | | | | | |
| Combined | | | | | | | | | | | | | |
| Mean | 3.78 | 3.89- | 3.24@# | 2.96@# | 3.82-- | 3.08@# | D | D | - | - | I | - | - |
| Std. Err. | 0.13 | 0.11 | 0.12 | 0.10 | 0.19 | 0.11 | | | | | | | |
| N | 20 | 18 | 20 | 20 | 18 | 19 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 6a: Bone Marrow-Erythroid Series / Femur x 10**6
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Metarubricyte (/ Femur x 10**6)** | | | | | | | | | | | | | |
| Male | | | | | | | | | | | | | |
| Mean | 0.53 | 0.51- | 0.12@# | 0.15@# | 0.12@# | 0.10@# | D | D | - | - | - | - | - |
| Std. Err. | 0.03 | 0.03 | 0.01 | 0.01 | 0.01 | 0.01 | | | | | | | |
| N | 10 | 10 | 10 | 10 | 10 | 10 | | | | | | | |
| .... | | | | | | | | | | | | | |
| Female | | | | | | | | | | | | | |
| Mean | 0.49 | 0.50- | 0.22@# | 0.10@# | 0.41-- | 0.34@# | D | D | * | - | I | I | * |
| Std. Err. | 0.05 | 0.05 | 0.02 | 0.01 | 0.04 | 0.02 | | | | | | | |
| N | 10 | 8 | 10 | 10 | 8 | 9 | | | | | | | |
| Combined | | | | | | | | | | | | | |
| Mean | 0.51 | 0.50 | 0.17 | 0.13 | 0.25 | 0.22 | | | | | | | |
| Std. Err. | 0.03 | 0.03 | 0.01 | 0.01 | 0.04 | 0.03 | | | | | | | |
| N | 20 | 18 | 20 | 20 | 18 | 19 | | | | | | | |
| **Total Erythroid (/ Femur x 10**6)** | | | | | | | | | | | | | |
| Male | | | | | | | | | | | | | |
| Mean | 4.30 | 4.55 | 3.69 | 3.34 | 4.41 | 3.39 | | | | | | | |
| Std. Err. | 0.23 | 0.15 | 0.15 | 0.12 | 0.20 | 0.11 | | | | | | | |
| N | 10 | 10 | 10 | 10 | 10 | 10 | | | | | | | |
| .... | | | | | | | | | | | | | |
| Female | | | | | | | | | | | | | |
| Mean | 4.29 | 4.21 | 3.12 | 2.84 | 3.65 | 3.18 | | | | | | | |
| Std. Err. | 0.20 | 0.22 | 0.15 | 0.12 | 0.25 | 0.17 | | | | | | | |
| N | 10 | 8 | 10 | 10 | 8 | 9 | | | | | | | |
| Combined | | | | | | | | | | | | | |
| Mean | 4.29 | 4.40- | 3.41@# | 3.09@# | 4.07-- | 3.29@# | D | D | - | - | I | - | - |
| Std. Err. | 0.15 | 0.13 | 0.12 | 0.10 | 0.18 | 0.10 | | | | | | | |
| N | 20 | 18 | 20 | 20 | 18 | 19 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 7: Bone Marrow-Megakaryocytic Series %
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Megakaryoblast (%)** | | | | | | | | | | | | | |
| Male | | | | | | | | | | | | | |
| Mean | 0.0 | 0.0 | 0.0 | 0.0 | 0.0 | 0.0 | | | | | | | |
| Std. Err. | 0.0 | 0.0 | 0.0 | 0.0 | 0.0 | 0.0 | | | | | | | |
| N | 10 | 10 | 10 | 10 | 10 | 10 | | | | | | | |
| .... | | | | | | | | | | | | | |
| Female | | | | | | | | | | | | | |
| Mean | 0.0 | 0.0 | 0.0 | 0.0 | 0.0 | 0.0 | | | | | | | |
| Std. Err. | 0.0 | 0.0 | 0.0 | 0.0 | 0.0 | 0.0 | | | | | | | |
| N | 10 | 8 | 10 | 10 | 8 | 9 | | | | | | | |
| Combined | | | | | | | | | | | | | |
| Mean | 0.0 | 0.0- | 0.0-- | 0.0-- | 0.0-- | 0.0-- | - | - | - | - | - | - | - |
| Std. Err. | 0.0 | 0.0 | 0.0 | 0.0 | 0.0 | 0.0 | | | | | | | |
| N | 20 | 18 | 20 | 20 | 18 | 19 | | | | | | | |
| **Promegakaryocyte (%)** | | | | | | | | | | | | | |
| Male | | | | | | | | | | | | | |
| Mean | 0.0 | 0.0 | 0.0 | 0.0 | 0.0 | 0.0 | | | | | | | |
| Std. Err. | 0.0 | 0.0 | 0.0 | 0.0 | 0.0 | 0.0 | | | | | | | |
| N | 10 | 10 | 10 | 10 | 10 | 10 | | | | | | | |
| .... | | | | | | | | | | | | | |
| Female | | | | | | | | | | | | | |
| Mean | 0.0 | 0.0 | 0.0 | 0.0 | 0.0 | 0.0 | | | | | | | |
| Std. Err. | 0.0 | 0.0 | 0.0 | 0.0 | 0.0 | 0.0 | | | | | | | |
| N | 10 | 8 | 10 | 10 | 8 | 9 | | | | | | | |
| Combined | | | | | | | | | | | | | |
| Mean | 0.0 | 0.0- | 0.0-- | 0.0-- | 0.0-- | 0.0-- | - | - | - | - | - | - | - |
| Std. Err. | 0.0 | 0.0 | 0.0 | 0.0 | 0.0 | 0.0 | | | | | | | |
| N | 20 | 18 | 20 | 20 | 18 | 19 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 7: Bone Marrow-Megakaryocytic Series %
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Megakaryocyte (%)** | | | | | | | | | | | | | |
| Male | | | | | | | | | | | | | |
| Mean | 0.0 | 0.1 | 0.0 | 0.0 | 0.1 | 0.0 | | | | | | | |
| Std. Err. | 0.0 | 0.0 | 0.0 | 0.0 | 0.0 | 0.0 | | | | | | | |
| N | 10 | 10 | 10 | 10 | 10 | 10 | | | | | | | |
| Female | | | | | | | | | | | | | |
| Mean | 0.1 | 0.1 | 0.1 | 0.1 | 0.1 | 0.1 | | | | | | | |
| Std. Err. | 0.0 | 0.0 | 0.0 | 0.0 | 0.0 | 0.0 | | | | | | | |
| N | 10 | 8 | 10 | 10 | 8 | 9 | | | | | | | |
| Combined | | | | | | | | | | | | | |
| Mean | 0.1 | 0.1- | 0.0-- | 0.1-- | 0.1-- | 0.0-- | - | - | - | - | - | - | - |
| Std. Err. | 0.0 | 0.0 | 0.0 | 0.0 | 0.0 | 0.0 | | | | | | | |
| N | 20 | 18 | 20 | 20 | 18 | 19 | | | | | | | |
| **Megakaryoblast (/ Femur x 10**6)** | | | | | | | | | | | | | |
| Male | | | | | | | | | | | | | |
| Mean | 0.000 | 0.000 | 0.000 | 0.000 | 0.000 | 0.000 | | | | | | | |
| Std. Err. | 0.000 | 0.000 | 0.000 | 0.000 | 0.000 | 0.000 | | | | | | | |
| N | 10 | 10 | 10 | 10 | 10 | 10 | | | | | | | |
| Female | | | | | | | | | | | | | |
| Mean | 0.000 | 0.000 | 0.000 | 0.000 | 0.000 | 0.000 | | | | | | | |
| Std. Err. | 0.000 | 0.000 | 0.000 | 0.000 | 0.000 | 0.000 | | | | | | | |
| N | 10 | 8 | 10 | 10 | 8 | 9 | | | | | | | |
| Combined | | | | | | | | | | | | | |
| Mean | 0.000 | 0.000- | 0.000-- | 0.000-- | 0.000-- | 0.000-- | - | - | - | - | - | - | - |
| Std. Err. | 0.000 | 0.000 | 0.000 | 0.000 | 0.000 | 0.000 | | | | | | | |
| N | 20 | 18 | 20 | 20 | 18 | 19 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 7a: Bone Marrow-Megakaryocytic Series / Femur x 10**6
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Promegakaryocyte (/ Femur x 10**6)** | | | | | | | | | | | | | |
| Male | | | | | | | | | | | | | |
| Mean | 0.000 | 0.000 | 0.000 | 0.000 | 0.000 | 0.000 | | | | | | | |
| Std. Err. | 0.000 | 0.000 | 0.000 | 0.000 | 0.000 | 0.000 | | | | | | | |
| N | 10 | 10 | 10 | 10 | 10 | 10 | | | | | | | |
| .... | | | | | | | | | | | | | |
| Female | | | | | | | | | | | | | |
| Mean | 0.000 | 0.000 | 0.000 | 0.000 | 0.000 | 0.000 | | | | | | | |
| Std. Err. | 0.000 | 0.000 | 0.000 | 0.000 | 0.000 | 0.000 | | | | | | | |
| N | 10 | 8 | 10 | 10 | 8 | 9 | | | | | | | |
| Combined | | | | | | | | | | | | | |
| Mean | 0.000 | 0.000- | 0.000-- | 0.000-- | 0.000-- | 0.000-- | - | - | - | - | - | - | - |
| Std. Err. | 0.000 | 0.000 | 0.000 | 0.000 | 0.000 | 0.000 | | | | | | | |
| N | 20 | 18 | 20 | 20 | 18 | 19 | | | | | | | |
| **Megakaryocyte (/ Femur x 10**6)** | | | | | | | | | | | | | |
| Male | | | | | | | | | | | | | |
| Mean | 0.002 | 0.010 | 0.002 | 0.004 | 0.007 | 0.002 | | | | | | | |
| Std. Err. | 0.002 | 0.004 | 0.002 | 0.003 | 0.004 | 0.002 | | | | | | | |
| N | 10 | 10 | 10 | 10 | 10 | 10 | | | | | | | |
| .... | | | | | | | | | | | | | |
| Female | | | | | | | | | | | | | |
| Mean | 0.010 | 0.009 | 0.007 | 0.006 | 0.004 | 0.006 | | | | | | | |
| Std. Err. | 0.004 | 0.005 | 0.003 | 0.003 | 0.003 | 0.003 | | | | | | | |
| N | 10 | 8 | 10 | 10 | 8 | 9 | | | | | | | |
| Combined | | | | | | | | | | | | | |
| Mean | 0.006 | 0.010- | 0.004-- | 0.005-- | 0.006-- | 0.004-- | - | - | - | - | - | - | - |
| Std. Err. | 0.002 | 0.003 | 0.002 | 0.002 | 0.002 | 0.002 | | | | | | | |
| N | 20 | 18 | 20 | 20 | 18 | 19 | | | | | | | |

a Indicates Difference From Group 1 : 0 mg/kg AZT, 0.0% Procysteine
\# Indicates Difference From Group 2 : 0 mg/kg AZT, 1.0% Procysteine
\- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 8: Bone Marrow-Miscellaneous Cells %

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT | 0 mg/kg | 0 mg/kg | 50 mg/kg | 500 mg/kg | 50 mg/kg | 500 mg/kg | AZT DR at % Procyst. | | | Procysteine DR at mg/kg AZT | | | |
|---|---|---|---|---|---|---|---|---|---|---|---|---|---|
| Procysteine | 0.0% | 1.0% | 0.0% | 0.0% | 1.0% | 1.0% | 0.0 | 1.0 | DIF | 0 | 50 | 500 | DIF |
| **Lymphocyte (%)** | | | | | | | | | | | | | |
| Male | | | | | | | | | | | | | |
| Mean | 0.9 | 1.0- | 0.4@# | 0.2@# | 0.3@# | 0.2@# | | | | - | - | - | - |
| Std. Err. | 0.1 | 0.1 | 0.1 | 0.1 | 0.1 | 0.1 | | | | | | | |
| N | 10 | 10 | 10 | 10 | 10 | 10 | | | | | | | |
| .... | | | | | | | | | | | | | |
| Female | | | | | | | | | | | | | |
| Mean | 1.0 | 1.2- | 0.5@# | 0.2@# | 1.1-- | 0.8-- | | | | - | I | I | - |
| Std. Err. | 0.2 | 0.1 | 0.1 | 0.1 | 0.2 | 0.2 | | | | | | | |
| N | 10 | 8 | 10 | 10 | 8 | 9 | | | | | | | |
| Combined | | | | | | | | | | | | | |
| Mean | 1.0 | 1.1 | 0.4 | 0.2 | 0.7 | 0.5 | D | D | - | | | | |
| Std. Err. | 0.1 | 0.1 | 0.1 | 0.1 | 0.1 | 0.1 | | | | | | | |
| N | 20 | 18 | 20 | 20 | 18 | 19 | | | | | | | |
| **Monocyte (%)** | | | | | | | | | | | | | |
| Male | | | | | | | | | | | | | |
| Mean | 0.0 | 0.0 | 0.1 | 0.1 | 0.1 | 0.2 | | | | | | | |
| Std. Err. | 0.0 | 0.0 | 0.0 | 0.0 | 0.1 | 0.0 | | | | | | | |
| N | 10 | 10 | 10 | 10 | 10 | 10 | | | | | | | |
| .... | | | | | | | | | | | | | |
| Female | | | | | | | | | | | | | |
| Mean | 0.1 | 0.1 | 0.1 | 0.1 | 0.1 | 0.1 | | | | | | | |
| Std. Err. | 0.1 | 0.1 | 0.0 | 0.0 | 0.0 | 0.0 | | | | | | | |
| N | 10 | 8 | 10 | 10 | 8 | 9 | | | | | | | |
| Combined | | | | | | | | | | | | | |
| Mean | 0.1 | 0.0- | 0.1-- | 0.1-- | 0.1-- | 0.1-- | - | - | - | - | - | - | - |
| Std. Err. | 0.0 | 0.0 | 0.0 | 0.0 | 0.0 | 0.0 | | | | | | | |
| N | 20 | 18 | 20 | 20 | 18 | 19 | | | | | | | |

@ Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 8: Bone Marrow-Miscellaneous Cells %

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT | 0 mg/kg | 0 mg/kg | 50 mg/kg | 500 mg/kg | 50 mg/kg | 500 mg/kg | AZT DR at % Procyst. | | | Procysteine DR at mg/kg AZT | | | |
|---|---|---|---|---|---|---|---|---|---|---|---|---|---|
| Procysteine | 0.0% | 1.0% | 0.0% | 0.0% | 1.0% | 1.0% | 0.0 | 1.0 | DIF | 0 | 50 | 500 | DIF |
| **Plasma Cell (%)** | | | | | | | | | | | | | |
| Male | | | | | | | | | | | | | |
| Mean | 0.2 | 0.0- | 0.3-- | 0.4@# | 0.4-# | 0.4-# | | | | | | | |
| Std. Err. | 0.0 | 0.0 | 0.1 | 0.1 | 0.1 | 0.1 | | | | | | | |
| N | 10 | 10 | 10 | 10 | 10 | 10 | | | | | | | |
| .... | | | | | | | | | | | | | |
| Female | | | | | | | | | | | | | |
| Mean | 0.1 | 0.3- | 0.1-- | 0.1-- | 0.3-- | 0.3@- | | | | | | | |
| Std. Err. | 0.0 | 0.1 | 0.0 | 0.0 | 0.1 | 0.1 | | | | | | | |
| N | 10 | 8 | 10 | 10 | 8 | 9 | | | | | | | |
| Combined | | | | | | | | | | | | | |
| Mean | 0.1 | 0.1 | 0.2 | 0.3 | 0.3 | 0.3 | - | I | - | - | - | - | - |
| Std. Err. | 0.0 | 0.1 | 0.0 | 0.0 | 0.0 | 0.1 | | | | | | | |
| N | 20 | 18 | 20 | 20 | 18 | 19 | | | | | | | |
| **Mast Cell (%)** | | | | | | | | | | | | | |
| Male | | | | | | | | | | | | | |
| Mean | 0.00 | 0.02 | 0.00 | 0.00 | 0.00 | 0.00 | | | | | | | |
| Std. Err. | 0.00 | 0.02 | 0.00 | 0.00 | 0.00 | 0.00 | | | | | | | |
| N | 10 | 10 | 10 | 10 | 10 | 10 | | | | | | | |
| .... | | | | | | | | | | | | | |
| Female | | | | | | | | | | | | | |
| Mean | 0.00 | 0.05 | 0.00 | 0.00 | 0.00 | 0.00 | | | | | | | |
| Std. Err. | 0.00 | 0.03 | 0.00 | 0.00 | 0.00 | 0.00 | | | | | | | |
| N. | 10 | 8 | 10 | 10 | 8 | 9 | | | | | | | |
| Combined | | | | | | | | | | | | | |
| Mean | 0.00 | 0.03@ | 0.00-# | 0.00-# | 0.00-# | 0.00-# | - | D | * | I | - | - | - |
| Std. Err. | 0.00 | 0.02 | 0.00 | 0.00 | 0.00 | 0.00 | | | | | | | |
| N | 20 | 18 | 20 | 20 | 18 | 19 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 8: Bone Marrow-Miscellaneous Cells %
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

Reticulum Cell (%)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Male | | | | | | | | | | | | | |
| Mean | 0.1 | 0.2- | 0.5-- | 0.4-- | 0.6@- | 0.5-- | | | | - | - | - | - |
| Std. Err. | 0.1 | 0.1 | 0.1 | 0.1 | 0.1 | 0.1 | | | | | | | |
| N | 10 | 10 | 10 | 10 | 10 | 10 | | | | | | | |
| .... | | | | | | | | | | | | | |
| Female | | | | | | | | | | | | | |
| Mean | 0.4 | 0.2- | 0.5-- | 0.8-# | 0.3-- | 0.1-- | | | | - | - | D | - |
| Std. Err. | 0.2 | 0.1 | 0.1 | 0.1 | 0.1 | 0.0 | | | | | | | |
| N | 10 | 8 | 10 | 10 | 8 | 9 | | | | | | | |
| Combined | | | | | | | | | | | | | |
| Mean | 0.3 | 0.2 | 0.5 | 0.6 | 0.4 | 0.3 | I | - | - | | | | |
| Std. Err. | 0.1 | 0.1 | 0.1 | 0.1 | 0.1 | 0.1 | | | | | | | |
| N | 20 | 18 | 20 | 20 | 18 | 19 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 8a: Bone Marrow-Miscellaneous Cells / Femur x 10**6

Listing of Means and Standard Errors; Comparison of Means to Group 1 and 2 Means

Descriptions and Comparisons of Dosage Response Effects

DR Indicates Dosage Response, i.e., Test of Slope; DIF Indicates Difference Between (Among) DR Effects

| AZT | 0 mg/kg | 0 mg/kg | 50 mg/kg | 500 mg/kg | 50 mg/kg | 500 mg/kg | AZT DR at % Procyst. | | | Procysteine DR at mg/kg AZT | | | |
|---|---|---|---|---|---|---|---|---|---|---|---|---|---|
| Procysteine | 0.0% | 1.0% | 0.0% | 0.0% | 1.0% | 1.0% | 0.0 | 1.0 | DIF | 0 | 50 | 500 | DIF |
| **Lymphocyte (/ Femur x 10**6)** | | | | | | | | | | | | | |
| Male | | | | | | | | | | | | | |
| Mean | 0.100 | 0.130 | 0.047 | 0.022 | 0.043 | 0.020 | | | | - | - | - | - |
| Std. Err. | 0.013 | 0.017 | 0.014 | 0.007 | 0.011 | 0.005 | | | | | | | |
| N | 10 | 10 | 10 | 10 | 10 | 10 | | | | | | | |
| .... | | | | | | | | | | | | | |
| Female | | | | | | | | | | | | | |
| Mean | 0.118 | 0.141 | 0.046 | 0.015 | 0.107 | 0.075 | | | | - | I | I | - |
| Std. Err. | 0.020 | 0.015 | 0.007 | 0.007 | 0.014 | 0.016 | | | | | | | |
| N | 10 | 8 | 10 | 10 | 8 | 9 | | | | | | | |
| Combined | | | | | | | | | | | | | |
| Mean | 0.109 | 0.135- | 0.047@# | 0.019@# | 0.072-# | 0.046@# | D | D | - | | | | |
| Std. Err. | 0.012 | 0.011 | 0.008 | 0.005 | 0.011 | 0.010 | | | | | | | |
| N | 20 | 18 | 20 | 20 | 18 | 19 | | | | | | | |
| **Monocyte (/ Femur x 10**6)** | | | | | | | | | | | | | |
| Male | | | | | | | | | | | | | |
| Mean | 0.005 | 0.003 | 0.011 | 0.008 | 0.018 | 0.016 | | | | | | | |
| Std. Err. | 0.003 | 0.003 | 0.004 | 0.003 | 0.009 | 0.005 | | | | | | | |
| N | 10 | 10 | 10 | 10 | 10 | 10 | | | | | | | |
| .... | | | | | | | | | | | | | |
| Female | | | | | | | | | | | | | |
| Mean | 0.008 | 0.006 | 0.006 | 0.008 | 0.009 | 0.006 | | | | | | | |
| Std. Err. | 0.005 | 0.006 | 0.003 | 0.005 | 0.004 | 0.003 | | | | | | | |
| N | 10 | 8 | 10 | 10 | 8 | 9 | | | | | | | |
| Combined | | | | | | | | | | | | | |
| Mean | 0.007 | 0.004- | 0.008-- | 0.008-- | 0.014-- | 0.012-- | - | - | - | - | - | - | - |
| Std. Err. | 0.003 | 0.003 | 0.002 | 0.003 | 0.005 | 0.003 | | | | | | | |
| N | 20 | 18 | 20 | 20 | 18 | 19 | | | | | | | |

@ Indicates Difference From Group 1 : 0 mg/kg AZT, 0.0% Procysteine

# Indicates Difference From Group 2 : 0 mg/kg AZT, 1.0% Procysteine

- Indicates Not Significant DR, DIF or Comparison to Group 1 or 2

D Indicates Decreasing DR

I Indicates Increasing DR

* Indicates Significant DIF at Alpha=0.01

For Combined Sexes, Blanks for DR and DIF or for Comparison to Group 1 or 2 Indicate Sexes Differ Therefore Tests Are Performed by Sex

For Each Sex, Blanks for DR and DIF or for Comparison to Group 1 or 2 Indicate Sexes Did Not Differ Therefore Tests Are Performed Across Sex

Table 8a: Bone Marrow-Miscellaneous Cells / Femur x 10**6
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

Plasma Cell (/ Femur x 10**6)

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Male | | | | | | | | | | | | | |
| Mean | 0.020 | 0.005- | 0.030-- | 0.045-# | 0.048a# | 0.037-# | | | | | | | |
| Std. Err. | 0.005 | 0.003 | 0.006 | 0.007 | 0.009 | 0.010 | | | | | | | |
| N | 10 | 10 | 10 | 10 | 10 | 10 | | | | | | | |
| .... | | | | | | | | | | | | | |
| Female | | | | | | | | | | | | | |
| Mean | 0.007 | 0.032- | 0.008-- | 0.013-- | 0.027-- | 0.029-- | | | | | | | |
| Std. Err. | 0.003 | 0.012 | 0.004 | 0.004 | 0.009 | 0.009 | | | | | | | |
| N | 10 | 8 | 10 | 10 | 8 | 9 | | | | | | | |
| Combined | | | | | | | | | | | | | |
| Mean | 0.013 | 0.017 | 0.019 | 0.029 | 0.038 | 0.033 | - | - | - | - | - | - | - |
| Std. Err. | 0.003 | 0.006 | 0.004 | 0.005 | 0.007 | 0.007 | | | | | | | |
| N | 20 | 18 | 20 | 20 | 18 | 19 | | | | | | | |

Mast Cell (/ Femur x 10**6)

| AZT<br>Procysteine | 0 mg/kg<br>0.0% | 0 mg/kg<br>1.0% | 50 mg/kg<br>0.0% | 500 mg/kg<br>0.0% | 50 mg/kg<br>1.0% | 500 mg/kg<br>1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Male | | | | | | | | | | | | | |
| Mean | 0.000 | 0.003 | 0.000 | 0.000 | 0.000 | 0.000 | | | | | | | |
| Std. Err. | 0.000 | 0.003 | 0.000 | 0.000 | 0.000 | 0.000 | | | | | | | |
| N | 10 | 10 | 10 | 10 | 10 | 10 | | | | | | | |
| .... | | | | | | | | | | | | | |
| Female | | | | | | | | | | | | | |
| Mean | 0.000 | 0.006 | 0.000 | 0.000 | 0.000 | 0.000 | | | | | | | |
| Std. Err. | 0.000 | 0.004 | 0.000 | 0.000 | 0.000 | 0.000 | | | | | | | |
| N | 10 | 8 | 10 | 10 | 8 | 9 | | | | | | | |
| Combined | | | | | | | | | | | | | |
| Mean | 0.000 | 0.004a | 0.000-# | 0.000-# | 0.000-# | 0.000-# | - | D | * | I | - | - | - |
| Std. Err. | 0.000 | 0.002 | 0.000 | 0.000 | 0.000 | 0.000 | | | | | | | |
| N | 20 | 18 | 20 | 20 | 18 | 19 | | | | | | | |

a Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 8a: Bone Marrow-Miscellaneous Cells / Femur x 10**6
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

Reticulum Cell (/ Femur x 10**6)

| AZT | 0 mg/kg | 0 mg/kg | 50 mg/kg | 500 mg/kg | 50 mg/kg | 500 mg/kg | AZT DR at % Procyst. | | | Procysteine DR at mg/kg AZT | | | |
|---|---|---|---|---|---|---|---|---|---|---|---|---|---|
| Procysteine | 0.0% | 1.0% | 0.0% | 0.0% | 1.0% | 1.0% | 0.0 | 1.0 | DIF | 0 | 50 | 500 | DIF |
| Male | | | | | | | | | | | | | |
| Mean | 0.012 | 0.021- | 0.062@- | 0.043-- | 0.068@# | 0.053-- | | | | - | - | - | - |
| Std. Err. | 0.007 | 0.010 | 0.017 | 0.014 | 0.008 | 0.015 | | | | | | | |
| N | 10 | 10 | 10 | 10 | 10 | 10 | | | | | | | |
| Female | | | | | | | | | | | | | |
| Mean | 0.043 | 0.017- | 0.056-- | 0.069-# | 0.022-- | 0.007-- | | | | - | - | D | - |
| Std. Err. | 0.018 | 0.009 | 0.015 | 0.012 | 0.011 | 0.005 | | | | | | | |
| N | 10 | 8 | 10 | 10 | 8 | 9 | | | | | | | |
| Combined | | | | | | | | | | | | | |
| Mean | 0.028 | 0.019 | 0.059 | 0.056 | 0.048 | 0.031 | - | - | - | | | | |
| Std. Err. | 0.010 | 0.007 | 0.011 | 0.009 | 0.009 | 0.010 | | | | | | | |
| N | 20 | 18 | 20 | 20 | 18 | 19 | | | | | | | |

Table 9: Bone Marrow-Myeloid:Erythroid Ratio
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| AZT | 0 mg/kg | 0 mg/kg | 50 mg/kg | 500 mg/kg | 50 mg/kg | 500 mg/kg | AZT DR at % Procyst. | | | Procysteine DR at mg/kg AZT | | | |
|---|---|---|---|---|---|---|---|---|---|---|---|---|---|
| Procysteine | 0.0% | 1.0% | 0.0% | 0.0% | 1.0% | 1.0% | 0.0 | 1.0 | DIF | 0 | 50 | 500 | DIF |
| | Myeloid:Erythroid Ratio | | | | | | | | | | | | |
| Male | | | | | | | | | | | | | |
| Mean | 1.56 | 1.59- | 2.09@# | 2.01@# | 1.74@# | 1.77@# | I | I | * | - | D | D | * |
| Std. Err. | 0.02 | 0.02 | 0.02 | 0.02 | 0.01 | 0.02 | | | | | | | |
| N | 10 | 10 | 10 | 10 | 10 | 10 | | | | | | | |
| Female | | | | | | | | | | | | | |
| Mean | 1.58 | 1.58- | 2.01@# | 2.10@# | 1.59-- | 1.58-- | I | - | * | - | D | D | * |
| Std. Err. | 0.02 | 0.02 | 0.02 | 0.02 | 0.02 | 0.02 | | | | | | | |
| N | 10 | 8 | 10 | 10 | 8 | 9 | | | | | | | |
| Combined | | | | | | | | | | | | | |
| Mean | 1.57 | 1.59 | 2.05 | 2.06 | 1.67 | 1.68 | | | | | | | |
| Std. Err. | 0.01 | 0.01 | 0.02 | 0.02 | 0.02 | 0.03 | | | | | | | |
| N | 20 | 18 | 20 | 20 | 18 | 19 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 10: Bone Marrow Cells Before Plating
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| | Nucleated Cells /Femur (X 10**6) | | | | | | | | | | | | | |
|---|---|---|---|---|---|---|---|---|---|---|---|---|---|---|
| AZT | 0 mg/kg | 0 mg/kg | 50 mg/kg | 500 mg/kg | 50 mg/kg | 500 mg/kg | AZT DR at % Procyst. | | | Procysteine DR at mg/kg AZT | | | |
| Procysteine | 0.0% | 1.0% | 0.0% | 0.0% | 1.0% | 1.0% | 0.0 | 1.0 | DIF | 0 | 50 | 500 | DIF |
| Male | | | | | | | | | | | | | |
| Mean | 11.8 | 12.8 | 11.7 | 10.5 | 12.6 | 10.0 | | | | | | | |
| Std. Err. | 0.6 | 0.4 | 0.4 | 0.4 | 0.5 | 0.4 | | | | | | | |
| N | 10 | 10 | 10 | 10 | 10 | 10 | | | | | | | |
| .... | | | | | | | | | | | | | |
| Female | | | | | | | | | | | | | |
| Mean | 11.4 | 12.0 | 9.9 | 8.8 | 10.4 | 9.0 | | | | | | | |
| Std. Err. | 0.5 | 0.6 | 0.5 | 0.4 | 0.6 | 0.4 | | | | | | | |
| N | 10 | 9 | 10 | 10 | 9 | 10 | | | | | | | |
| Combined | | | | | | | | | | | | | |
| Mean | 11.6 | 12.4- | 10.8-# | 9.6a# | 11.5-- | 9.5a# | D | D | - | - | - | - | - |
| Std. Err. | 0.4 | 0.3 | 0.4 | 0.3 | 0.5 | 0.3 | | | | | | | |
| N | 20 | 19 | 20 | 20 | 19 | 20 | | | | | | | |

a Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 11: Colony Forming Units/10**4 Cells Plated
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | AZT DR at % Procyst. 1.0 | AZT DR at % Procyst. DIF | Procysteine DR at mg/kg AZT 0 | Procysteine DR at mg/kg AZT 50 | Procysteine DR at mg/kg AZT 500 | Procysteine DR at mg/kg AZT DIF |
|---|---|---|---|---|---|---|---|---|---|---|---|---|---|
| **Myeloid (No. of Colonies/10**4 Cells Plated)** | | | | | | | | | | | | | |
| Male | | | | | | | | | | | | | |
| Mean | 35.6 | 33.9 | 32.6 | 40.7 | 37.6 | 42.2 | | | | | | | |
| Std. Err. | 1.8 | 1.4 | 2.5 | 2.6 | 1.7 | 3.3 | | | | | | | |
| N | 10 | 10 | 10 | 10 | 10 | 10 | | | | | | | |
| .... | | | | | | | | | | | | | |
| Female | | | | | | | | | | | | | |
| Mean | 34.9 | 32.3 | 35.5 | 46.8 | 31.7 | 43.4 | | | | | | | |
| Std. Err. | 1.8 | 1.6 | 1.8 | 2.7 | 2.3 | 2.4 | | | | | | | |
| N | 10 | 9 | 10 | 10 | 9 | 10 | | | | | | | |
| Combined | | | | | | | | | | | | | |
| Mean | 35.2 | 33.1- | 34.0-- | 43.8@# | 34.8-- | 42.8@# | I | I | - | - | - | - | - |
| Std. Err. | 1.3 | 1.0 | 1.5 | 2.0 | 1.5 | 2.0 | | | | | | | |
| N | 20 | 19 | 20 | 20 | 19 | 20 | | | | | | | |
| **Erythroid (No. of Colonies/10**4 Cells Plated)** | | | | | | | | | | | | | |
| Male | | | | | | | | | | | | | |
| Mean | 3.8 | 3.1 | 3.6 | 4.1 | 4.2 | 4.2 | | | | | | | |
| Std. Err. | 0.5 | 0.3 | 0.7 | 0.5 | 0.5 | 0.7 | | | | | | | |
| N | 10 | 10 | 10 | 10 | 10 | 10 | | | | | | | |
| .... | | | | | | | | | | | | | |
| Female | | | | | | | | | | | | | |
| Mean | 3.2 | 3.1 | 3.6 | 4.1 | 3.3 | 3.8 | | | | | | | |
| Std. Err. | 0.3 | 0.4 | 0.4 | 0.6 | 0.5 | 0.4 | | | | | | | |
| N | 10 | 9 | 10 | 10 | 9 | 10 | | | | | | | |
| Combined | | | | | | | | | | | | | |
| Mean | 3.5 | 3.1- | 3.6-- | 4.1-- | 3.8-- | 4.0-- | - | - | - | - | - | - | - |
| Std. Err. | 0.3 | 0.2 | 0.4 | 0.4 | 0.3 | 0.4 | | | | | | | |
| N | 20 | 19 | 20 | 20 | 19 | 20 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 11: Colony Forming Units/10**4 Cells Plated
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| AZT | 0 mg/kg | 0 mg/kg | 50 mg/kg | 500 mg/kg | 50 mg/kg | 500 mg/kg | AZT DR at % Procyst. | | | Procysteine DR at mg/kg AZT | | | |
|---|---|---|---|---|---|---|---|---|---|---|---|---|---|
| Procysteine | 0.0% | 1.0% | 0.0% | 0.0% | 1.0% | 1.0% | 0.0 | 1.0 | DIF | 0 | 50 | 500 | DIF |
| **Mixed (No. of Colonies/10**4 Cells Plated)** | | | | | | | | | | | | | |
| Male | | | | | | | | | | | | | |
| Mean | 0.1 | 0.1 | 0.0 | 0.0 | 0.0 | 0.1 | | | | | | | |
| Std. Err. | 0.1 | 0.1 | 0.0 | 0.0 | 0.0 | 0.0 | | | | | | | |
| N | 10 | 10 | 10 | 10 | 10 | 10 | | | | | | | |
| .... | | | | | | | | | | | | | |
| Female | | | | | | | | | | | | | |
| Mean | 0.2 | 0.3 | 0.2 | 0.1 | 0.2 | 0.1 | | | | | | | |
| Std. Err. | 0.2 | 0.1 | 0.1 | 0.1 | 0.1 | 0.0 | | | | | | | |
| N | 10 | 9 | 10 | 10 | 9 | 10 | | | | | | | |
| Combined | | | | | | | | | | | | | |
| Mean | 0.2 | 0.2- | 0.1-- | 0.1-- | 0.1-- | 0.1-- | - | - | - | - | - | - | - |
| Std. Err. | 0.1 | 0.1 | 0.0 | 0.0 | 0.1 | 0.0 | | | | | | | |
| N | 20 | 19 | 20 | 20 | 19 | 20 | | | | | | | |
| **Total (No. of Colonies/10**4 Cells Plated)** | | | | | | | | | | | | | |
| Male | | | | | | | | | | | | | |
| Mean | 39.5 | 37.1 | 36.2 | 44.9 | 41.9 | 46.5 | | | | | | | |
| Std. Err. | 1.9 | 1.4 | 2.5 | 2.8 | 1.7 | 3.3 | | | | | | | |
| N | 10 | 10 | 10 | 10 | 10 | 10 | | | | | | | |
| .... | | | | | | | | | | | | | |
| Female | | | | | | | | | | | | | |
| Mean | 38.3 | 35.7 | 39.3 | 50.9 | 35.2 | 47.3 | | | | | | | |
| Std. Err. | 1.7 | 1.6 | 1.8 | 2.9 | 2.2 | 2.5 | | | | | | | |
| N | 10 | 9 | 10 | 10 | 9 | 10 | | | | | | | |
| Combined | | | | | | | | | | | | | |
| Mean | 38.9 | 36.4- | 37.7-- | 47.9@# | 38.7-- | 46.9@# | I | I | - | - | - | - | - |
| Std. Err. | 1.2 | 1.0 | 1.5 | 2.1 | 1.6 | 2.0 | | | | | | | |
| N | 20 | 19 | 20 | 20 | 19 | 20 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 11a: Colony Forming Units/Femur
Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 1.0 DIF | Procysteine DR at mg/kg AZT 0 50 500 DIF |
|---|---|---|---|---|---|---|---|---|
| **Myeloid (No. of Colonies/Femur)** | | | | | | | | |
| Male | | | | | | | | |
| Mean | 42307 | 43463 | 37791 | 42343 | 47641 | 41598 | | |
| Std. Err. | 3507 | 2652 | 2909 | 2405 | 3163 | 2702 | | |
| N | 10 | 10 | 10 | 10 | 10 | 10 | | |
| .... | | | | | | | | |
| Female | | | | | | | | |
| Mean | 39939 | 38527 | 35617 | 40695 | 33137 | 39265 | | |
| Std. Err. | 3104 | 2590 | 3126 | 2271 | 3373 | 3068 | | |
| N | 10 | 9 | 10 | 10 | 9 | 10 | | |
| Combined | | | | | | | | |
| Mean | 41123 | 41125- | 36704-- | 41519-- | 40771-- | 40432-- | - - - | - - - - |
| Std. Err. | 2295 | 1898 | 2093 | 1621 | 2818 | 2008 | | |
| N | 20 | 19 | 20 | 20 | 19 | 20 | | |
| **Erythroid (No. of Colonies/Femur)** | | | | | | | | |
| Male | | | | | | | | |
| Mean | 4705 | 3937 | 4034 | 4414 | 5414 | 4253 | | |
| Std. Err. | 746 | 405 | 792 | 564 | 654 | 747 | | |
| N | 10 | 10 | 10 | 10 | 10 | 10 | | |
| .... | | | | | | | | |
| Female | | | | | | | | |
| Mean | 3672 | 3701 | 3570 | 3443 | 3460 | 3490 | | |
| Std. Err. | 369 | 447 | 385 | 536 | 633 | 506 | | |
| N | 10 | 9 | 10 | 10 | 9 | 10 | | |
| Combined | | | | | | | | |
| Mean | 4189 | 3825- | 3802-- | 3929-- | 4488-- | 3872-- | - - - | - - - - |
| Std. Err. | 422 | 293 | 432 | 395 | 500 | 448 | | |
| N | 20 | 19 | 20 | 20 | 19 | 20 | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 11a: Colony Forming Units/Femur

Listing of Means and Standard Errors; Comparison of Means to Group 1 and 2 Means
Descriptions and Comparisons of Dosage Response Effects
DR Indicates Dosage Response, i.e., Test of Slope; DIF Indicates Difference Between (Among) DR Effects

Mixed (No. of Colonies/Femur)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Male** | | | | | | | | | | | | | |
| Mean | 170 | 125 | 0 | 40 | 50 | 63 | | | | | | | |
| Std. Err. | 125 | 64 | 0 | 40 | 50 | 42 | | | | | | | |
| N | 10 | 10 | 10 | 10 | 10 | 10 | | | | | | | |
| .... | | | | | | | | | | | | | |
| **Female** | | | | | | | | | | | | | |
| Mean | 317 | 317 | 182 | 91 | 202 | 52 | | | | | | | |
| Std. Err. | 228 | 180 | 83 | 47 | 121 | 34 | | | | | | | |
| N | 10 | 9 | 10 | 10 | 9 | 10 | | | | | | | |
| **Combined** | | | | | | | | | | | | | |
| Mean | 243 | 216- | 91-- | 66-- | 122-- | 58-- | - | - | - | - | - | - | - |
| Std. Err. | 128 | 92 | 45 | 31 | 64 | 27 | | | | | | | |
| N | 20 | 19 | 20 | 20 | 19 | 20 | | | | | | | |

Total (No. of Colonies/Femur)

| AZT / Procysteine | 0 mg/kg 0.0% | 0 mg/kg 1.0% | 50 mg/kg 0.0% | 500 mg/kg 0.0% | 50 mg/kg 1.0% | 500 mg/kg 1.0% | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| **Male** | | | | | | | | | | | | | |
| Mean | 47182 | 47525 | 41824 | 46797 | 53105 | 45914 | | | | | | | |
| Std. Err. | 3976 | 2790 | 2753 | 2774 | 3471 | 2662 | | | | | | | |
| N | 10 | 10 | 10 | 10 | 10 | 10 | | | | | | | |
| .... | | | | | | | | | | | | | |
| **Female** | | | | | | | | | | | | | |
| Mean | 43928 | 42544 | 39369 | 44229 | 36799 | 42807 | | | | | | | |
| Std. Err. | 3254 | 2638 | 3298 | 2291 | 3536 | 3378 | | | | | | | |
| N | 10 | 9 | 10 | 10 | 9 | 10 | | | | | | | |
| **Combined** | | | | | | | | | | | | | |
| Mean | 45555 | 45166- | 40596-- | 45513-- | 45381-- | 44361-- | - | - | - | - | - | - | - |
| Std. Err. | 2528 | 1964 | 2109 | 1775 | 3080 | 2123 | | | | | | | |
| N | 20 | 19 | 20 | 20 | 19 | 20 | | | | | | | |

@ Indicates Difference From Group 1 : 0 mg/kg AZT, 0.0% Procysteine
\# Indicates Difference From Group 2 : 0 mg/kg AZT, 1.0% Procysteine
\- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

Table 12: Gross Liver Findings
Listing of Counts for Animals in Blocks 2-5; Comparison of Counts to Group 1 and 2 Counts
Descriptions and Comparisons of Dosage Response Effects
DR Indicates Dosage Response, i.e., Test of Slope; DIF Indicates Difference Between (Among) DR Effects

| AZT | 0 mg/kg | 0 mg/kg | 50 mg/kg | 500 mg/kg | 50 mg/kg | 500 mg/kg | AZT DR at % Procyst. 0.0 | 1.0 | DIF | Procysteine DR at mg/kg AZT 0 | 50 | 500 | DIF |
|---|---|---|---|---|---|---|---|---|---|---|---|---|---|
| Procysteine | 0.0% | 1.0% | 0.0% | 0.0% | 1.0% | 1.0% | | | | | | | |
| | Lesion Incidence | | | | | | | | | | | | |
| Male | | | | | | | | | | | | | |
| Count | 4 | 5 | 2 | 4 | 2 | 5 | | | | | | | |
| N | 8 | 8 | 8 | 8 | 8 | 8 | | | | | | | |
| .... | | | | | | | | | | | | | |
| Female | | | | | | | | | | | | | |
| Count | 0 | 2 | 0 | 5 | 3 | 3 | | | | | | | |
| N | 8 | 7 | 8 | 8 | 7 | 8 | | | | | | | |
| Combined | | | | | | | | | | | | | |
| Count | 4 | 7- | 2-- | 9-- | 5-- | 8-- | - | - | - | - | - | - | - |
| N | 16 | 15 | 16 | 16 | 15 | 16 | | | | | | | |

@ Indicates Difference From Group 1 : 0 mg/kg AZT, 0.0% Procysteine
# Indicates Difference From Group 2 : 0 mg/kg AZT, 1.0% Procysteine
- Indicates Not Significant DR, DIF or Comparison to Group 1 or 2
D Indicates Decreasing DR
I Indicates Increasing DR
* Indicates Significant DIF at Alpha=0.01
For Combined Sexes, Blanks for DR and DIF or for Comparison to Group 1 or 2 Indicate Sexes Differ Therefore Tests Are Performed by Sex
For Each Sex, Blanks for DR and DIF or for Comparison to Group 1 or 2 Indicate Sexes Did Not Differ Therefore Tests Are Performed Across Sex

## Results and Analysis

Terminal blood was evaluated for hematological effects, and bone marrow smears and aspirates were counted and $10^4$ nucleated cells of the aspirate were cultured 14 days to assess hematopoietic progenitor cells. Anemia, evidenced by significant ($p<0.01$) decreases in total hemoglobin concentration, hematocrit and erythrocyte counts, and increases in mean corpuscular volume, mean corpuscular hemoglobin and the incidence of polychromasia, anisocytosis, macrocytes and Howell-Jolly bodies, was similarly observed across both sexes for AZT and AZT/Procysteine™ -treated groups.

Bone marrow aspirates exhibited a significant reduction in nucleated cells for AZT and high-dose AZT/Procysteine™-treated groups compared to controls. No group differences were noted in burst forming units-erythroid for marrow cultures, however, a significant induction was observed in total colonies and colony forming units-granulocyte/macrophage for AZT and AZT/Procysteine™ groups.

Microscopic analysis of the bone marrow smears was conducted by evaluating 500 cells for the differential analysis and 500 cells for the myeloid: erythroid ratio.

Bone marrow smears of AZT-treated mice had a reduced total count of erythroid series cells due to a marked reduction of metarubricytes (nonproliferating) and a mild reduction of rubriblasts. AZT/Procysteine™-treated mice had a significantly less severe decrease in erythroid counts than did the AZT-only treated animals, with the rubriblast, rubricyte and metarubricyte (female) counts being similar to control values.

AZT significantly increased the proliferating compartment of the myeloid series, with a reduction in the nonproliferating compartment. The AZT/Procysteine™- treated mice had a less severe increase in the proliferating myeloid compartment compared to the AZT-only treated mice. The myeloid:erythroid ratios for AZT groups were significantly increased whereas those for AZT/Procysteine™ groups were at control level.

In conclusion, these results indicate that the coadministration of Procysteine™ with AZT should reduce at

least some indices of AZT-induced hematotoxicity.

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. Use in the manufacture of a pharmaceutical preparation of a composition that stimulates the intracellular synthesis of glutathione for the purpose of reducing toxicity associated with antiretroviral therapy in a patient receiving antiretroviral therapy.

2. Use in the manufacture of a pharmaceutical preparation of a composition that stimulates the intracellular synthesis of glutathione, for the purpose of ameliorating the toxicity of an antiretroviral compound administered to a patent in the treatment of AIDS.

3. Use according to Claim 1 or 2 in the manufacture of a parenterally administrable pharmaceutical preparation.

4. Use according to Claim 1 or 2 in the manufacture of an enterally administrable pharmaceutical preparation.

5. Use according to Claim 1, 2, 3 or 4 wherein the composition includes L-2-oxothiazolidine-4-carboxylate.

6. Use according to Claim 1, 2, 3 or 4, wherein the composition includes a glutathione ester.

7. Use according to Claim 1, 2, 3 or 4, wherein the composition includes a thiazolidine-4-carboxylate analog.

8. Use according to Claim 1, 2, 3 or 4, wherein the composition includes an ester chosen from the group consisting of a methyl ester of glutathione; and ethyl ester of glutathione; short chain acetyl esters of glutathione; and a glutathione isopropyl ester.

9. Use according to any preceding claim wherein the antiretroviral therapy comprises administration of AZT.

10. Use according to any one of Claims 1 to 8 wherein the antiretroviral therapy comprises administrating DDI.

11. Use according to any preceding claim wherein the composition includes proteins rich in cysteine.

12. Use according to Claim 11 wherein the proteins rich in cysteine are chosen from the group consisting of whey, egg white, serum albumin, and lactalbumin.

13. Use in the manufacture of a pharmaceutical preparation of L-2-oxothiazolidine-4-carboxylate for the purpose of reducing the toxicity of AZT in a patient receiving AZT therapy.

14. Use in the manufacture of a pharmaceutical preparation, having at least first and second separately administrable components, of an antiretroviral compound in one component and a composition in the other component that stimulates in a patient the intracellular synthesis of glutathione, for the purpose of treating a patient who is HIV positive.

15. Use according to Claim 14, wherein the antiretroviral compound is AZT and the composition is L-2-oxothiazolidine-4-carboxylate.

16. A pharmaceutical preparation having at least first and second separately administrable components, one including an antiretroviral compound and the other component including a composition which stimulates the intracellular synthesis of glutathione, when administered to a patient.

17. A pharmaceutical preparation according to Claim 16, wherein the composition is L-2-oxothiazolidine-4-carboxylate, or a thiozolidine-4-carboxylate analog.

18. A pharmaceutical preparation according to Claim 16, wherein the composition is a glutathione ester.

19. A pharmaceutical preparation according to Claim 16, 17 or 18, wherein the antiretroviral component is AZT, or DDI.

20. A pharmaceutical preparation according to any one of Claims 16 to 19, wherein the composition includes proteins rich in cysteine.

21. A pharmaceutical preparation according to Claim 20 wherein the proteins are chosen from whey, egg white, serum albumin and lactalbumin.